# EUROPEAN PATENT SPECIFICATION

(11) **EP 3 654 912 B1**
(45) Date of publication and mention of the grant of the patent: **17.02.2021**
(21) Application number: 17742426.4
(22) Date of filing: 20.07.2017
(51) Int. Cl.: A61J 1/20

(54) **DRUG MIXING DEVICE**
VORRICHTUNG ZUM MISCHEN VON ARZNEIMITTELN
DISPOSITIF DE MÉLANGE DE MÉDICAMENT

(43) Date of publication of application: 27.05.2020
(73) Proprietor: Janssen Biotech, Inc., Horsham, PA 19044 (US)
(72) Inventor: TASHJIAN, Paul, Malvern PA 19355 (US)
(74) Representative: Brunner, John Michael Owen
(86) International application number: PCT/EP2017/068416
(87) International publication number: WO 2019/015770

(56) References cited:
- WO-A1-93/11709
- WO-A1-2011/101351
- US-A1- 2002 007 671
- US-A1- 2006 245 980

## Description

### Technical Field

The present invention lies generally in the technical field of drug mixing devices and more specifically in the field of drug mixing devices for the reconstitution of a drug, prior to the drug's administration to a patient.

### Background

Drug administration to human or animal patients occurs daily in modern health and veterinary care. A particularly common form of drug administration is administration via a syringe, whereby a drug is injected into a patient.

Prior to administration the drug must be prepared. Whilst some drugs are able to be stored long term in a state suitable for administration, certain drugs require preparation immediately before use, which involves mixing a first component of the drug to be mixed with the second component of the drug to be mixed, in order to form a mixed drug. The first and second components may be fluid or solid, but once mixed form a fluid that may be administered to a patient, for example by a syringe.

The typical preparation steps of a mixed drug includes pouring fluid from a first container into a second container, where inside the second container is a powdered drug. Once poured, the fluid and the powdered drug mix to form the administrable drug. A syringe is then used to draw out the mixed drug from the container for later administration. One example of a drug that has been mixed prior to administration in this manner is Remicade (RTM) by Janssen Biotech, Inc. also known as infliximab, in which sterilised water is combined with powdered Remicade (RTM) in order to form the fluid for administration. Administration of Remicade (RTM) is used in the treatment of Crohn's disease and rheumatoid arthritis.

The preparation steps outlined above require a degree of skill by the user. The user must combine the components of the drug to be mixed in the correct order and then quickly administer them to the patient with the syringe. The preparation requires a series of manual manipulations requiring a high level of dexterity by the user and take significant time and are prone to error. In addition, a variety of potential hazards to the user arise from this process, for example the risk of needle sticks or spillage from the containers.

Several problems may also arise for the patient. Residual mixed drug may be left behind in the container when the drug is withdrawn into the syringe. The mixed drug may not be completely mixed prior to administration if administration is completed in haste, not least because it is difficult for the user to determine when mixing is complete. Furthermore, the mixing process may result in foaming or agglutination of the components, which restricts their clinical effectiveness.

WO 93/11709A1 describes a medication dispenser that is used to directly fill a syringe with measured amounts of one or more liquid medications, typically two different types of insulin, from containers, such as vials and cartridges each having a septum at one end. Each cartridge has a pierceable piston at the other end. The septum of each container is pierced by hollow liquid spikes while hollow gas spikes pierce the septum of the vial and the piston of the cartridge. Liquid is pumped out of the container and air is replaced into the container through the liquid and gas spikes. Two of the cartridges can contain a diluent and a lyophilized component respectively. The diluent in the first cartridge can be pumped into the second cartridge through a one-way valve to create a mixed pharmaceutical which is then pumped into the syringe, with or without another pharmaceutical.

A need exists for a safe, quick and easy-to-use drug mixing device that is compact and is compatible with conventional drug administration devices, but that can ensure complete mixing of the drug prior to administration. The device should also avoid potential hazards to the patient and to the user. In addition, the mixing device should be optimised for achieving the above goals with minimum wastage of drug. Furthermore, the drug mixing device should not rely on skilled health practitioners in order to be able to be used.

### Summary

The first aspect of the present invention relates to a drug mixing device. The drug mixing device comprises a movable actuator, wherein movement of the actuator causes mixing of a drug within the drug mixing device, and an elastic member coupled to the actuator, the elastic member configured upon release to transition from an extended state to a non-extended state, the elastic member storing energy in the extended state. The movement of the actuator is driven by the release of the stored energy as the elastic member transitions from the extended state towards the non-extended state. By this mechanism, the drug mixing device includes a self-contained source of work (energy) for causing mixing of the drug where the extent of the extended and non-extended states affords control over the amount stored. Furthermore, the space required for a bottomed out elastic member is not needed above the movable actuator.

In some embodiments, the movement of the actuator causes movement of a first component of the drug to be mixed from a first portion of the drug mixing device to a second portion of the drug mixing device where it is mixed with a second component to form the mixed drug, thereby the initially separated components are brought to the same location to initiate the mixing process.

In some embodiments, the mixing device further comprises a fluid driver. The fluid driver may comprise a movable piston and a driving fluid container. The container may comprise a container of driving fluid, wherein movement of the piston causes at least part of the driving fluid to be expelled from the driving fluid container. The volume of driving fluid expelled from the driving fluid container may lie between 1ml and 20ml.

In some embodiments, the elastic member comprises a spring. The spring may comprises a flat spring. Alternatively, the spring may comprise a coil spring.

The spring may comprise an extended portion, the extended portion arranged to substantially conform to an external contour of at least a portion of the actuator, the profile of the extended portion minimising the space required within the drug mixing device and ultimately permitting a smaller device.

The spring may further comprise a constant force spring, providing a consistent force for the driving movement of the actuator allowing a constant rate of mixing.

In embodiments comprising a coil spring, the coil spring may be in part or in its entirety wrapped around at least a portion of the actuator. The coil spring may be in part or its entirety wrapped around the driving fluid container. By wrapping the spring around at least a portion of the actuator, the hole in the centre of the spring may be effectively used and does not become 'dead space' within the device.

In some embodiments, the movement of the actuator is directed substantially towards a point to which the elastic member is secured.

In some embodiments, the mixing device further comprises a locking mechanism configured in a locked state to prevent the release of energy from the elastic member, and in an unlocked state to allow the release of energy from the elastic member. This protective safety feature enables the device to be triggered to release energy at the user's will.

The drug mixing device may be for reconstitution of the drug. The drug may be Remicade (RTM).

In some embodiments, the mixing device may comprise a housing. The housing may be configured to detachably receive within a first portion of the housing a first container for holding a first component of the drug to be mixed, and detachably receive within a second portion of the housing a second container for holding a second component of the drug to be mixed. Thus the device and containers may be provided as a kit to be assembled immediately prior to use.

The housing may be arranged such that, once received, the first and second containers are located in an opposing relationship. The opposing relationship provides the opportunity for a compact configuration of the drug mixing device.

In some embodiments, the first container comprises a first opening, the second container comprises a second opening, and the first and second openings oppose each other when the first and second containers are located within the housing.

The first container may comprise a closure on the first opening, and the second container comprises a closure on the second opening. At least one of the closures may comprise a septum that seals the container until penetrated/pierced by a needle or similar.

In some embodiments, the mixing device further comprises a transfer member configured, in use, to fluidly couple the first and second containers, wherein the transfer member is also configured to extend into at least one of the first container and the second container when the containers are received in the housing. The transfer member may be configured to extend through at least one of the closures of the first and second containers. The transfer member may comprise one or more pointed ends configured, in use, to pierce at least one of the first and second containers when the containers are received in the housing. The pointed end is configured to pierce the closure of at least one of the first and second containers.

In some embodiments, the fluid driver comprises a driving fluid transfer member and wherein the driving fluid transfer member is configured to fluidly couple the fluid driver to the first container, and the driving fluid transfer member is configured, in use, to extend into the first container.

In further embodiments, the driving fluid transfer member and the transfer member are configured, in use, to extend through the same surface of the first container when the first container is fluidly coupled to the fluid driver, facilitating a compact configuration and providing a single surface where fluid couplings must be established. By this arrangement on pushing the container into the housing, both transfer member and driving fluid transfer member pierce any closure on the container during the same pushing movement.

In some embodiments, the volume of at least one of the first container and the second container is in the range 1ml to 1000ml.

### Brief Description of the Figures

The present invention is described below with reference to the following figures, in which:
Figure 1 is a front perspective view of a drug mixing device according to an embodiment of the invention.
Figure 2 is a rear perspective view of the drug mixing device of Figure 1.
Figure 3 is a front view of the drug mixing device of Figure 1, with the pin and vent cap removed.
Figure 4 is a partial cutaway view of the drug mixing device of Figure 1, with half of the outer housing removed and a single container inserted.
Figure 5 is a cutaway view of the drug mixing device of Figure 1, with half of the outer housing and half of the inner support removed and no vials inserted.
Figure 6 is an exploded view of the drug mixing device of Figure 1.
Figure 7 is a cutaway view of the drug mixing device of Figure 1, with the insertion path of the two containers shown.
Figure 8 is a cutaway view of the drug mixing device of Figure 1, as shown in Figure 7, with the two containers fully inserted and the device is in the locked state.
Figure 9 is a cutaway view of the drug mixing device as shown in Figure 9, showing removal of the pin to place the device in an unlocked state.
Figure 10A is a cutaway view of the drug mixing device of Figure 1 during the initial stage of the drug mixing process.
Figure 10B is a cutaway view of the drug mixing device of Figure 1 during the final stage of drug mixing process.
Figure 11 is a front cutaway view of a fluid transfer assembly including the drug mixing device of Figure 1 and a drug administration device.
Figure 12 is a partial front cutaway view of the fluid assembly of Figure 11.
Figures 13A to F are side perspective views of the fluid transfer assembly comprising the drug mixing device of Figure 1 and a drug administration device.
Figures 14A to C are side views of a container and the transfer members of the drug mixing device of Figure 1.
Figure 15 is a side view of a container including a transfer member during dispensing of a fluid into the container.
Figure 16 is an exploded view of an exemplary locking mechanism of the drug mixing device of Figure 1.
Figure 17 is a partial cutaway view of a first type of detail of a transfer member of the drug mixing device of Figure 1.
Figure 18 is a partial cutaway view of a second type of detail of a transfer member of the drug mixing device of Figure 1.
Figures 19A to D are side views of a details of alternative transfer members of the drug mixing device of Figure 1.
Figures 20A to C are side views showing the insertion of a container into a port of the drug mixing device of Figure 1.
Figure 21A shows a cutaway view of an alternative actuator and locking mechanism able to be integrated into the mixing device of Figure 1. The locking mechanism is in the locked state.
Figure 21B is a cutaway view of the actuator and locking mechanism of figure 21A with the locking mechanism in the unlocked state.
Figure 22A is a cutaway view of an alternative actuator and locking mechanism able to be integrated into the mixing device of Figure 1. The locking mechanism is in the locked state.
Figure 22B is a cutaway view of the actuator and locking mechanism of figure 22A with the locking mechanism in the unlocked state.

### Detailed Disclosure

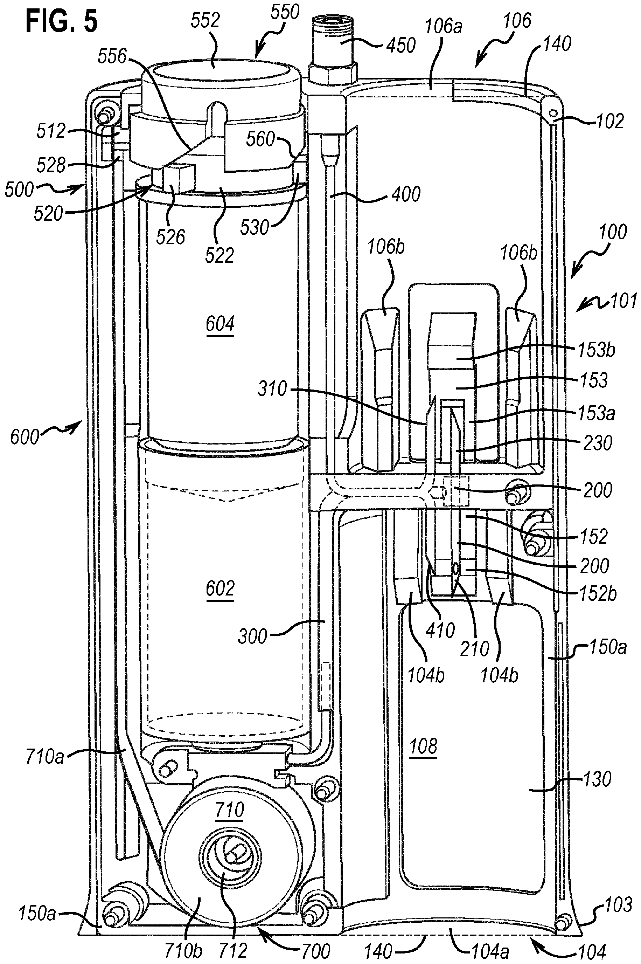

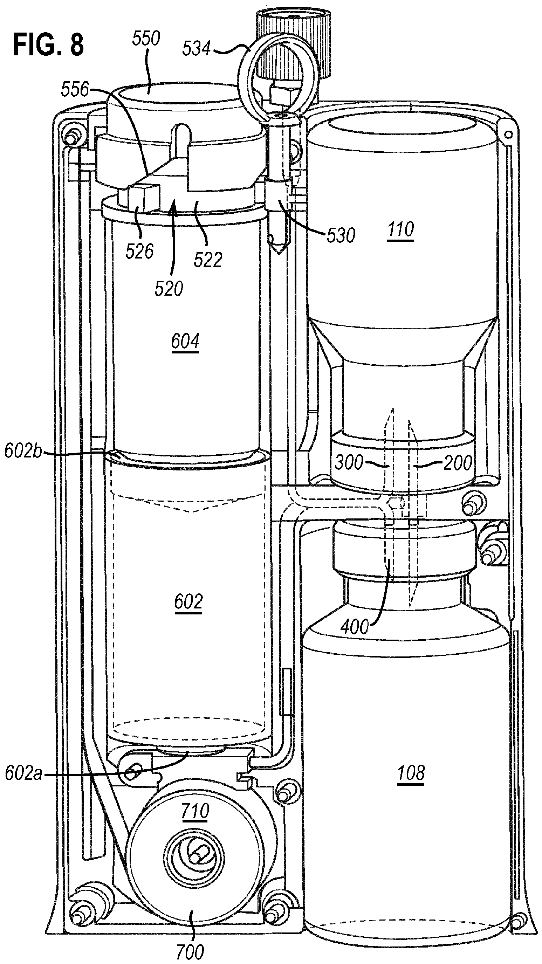

The following detailed disclosure outlines the features of one specific embodiment of the present invention. In addition, some (but by no means all) variants of the specific embodiment that might be implemented whilst still falling under the scope of the present invention are also described. Whilst the following description is subdivided into sections in order to aid the skilled person's comprehension, the specific substructure of the detailed description should not be seen as delimiting individual embodiments of the invention. On the contrary, features of the various sections may be combined as appropriate. For example, the flanged base 103 described in the housing and structure section may be combined in a device with the pressure driven mixing provided by a piston 604 and reservoir 602, as is shown in Figure 5. As an alternative example, the opposed configuration of the two vials 108 and 110 described in the housing and structure section may be included in an embodiment featuring a push and forget actuation mechanism. As a further illustrative example, the drawdown mechanism may be included in an embodiment featuring the staggered needles, as shown in Figure 8.

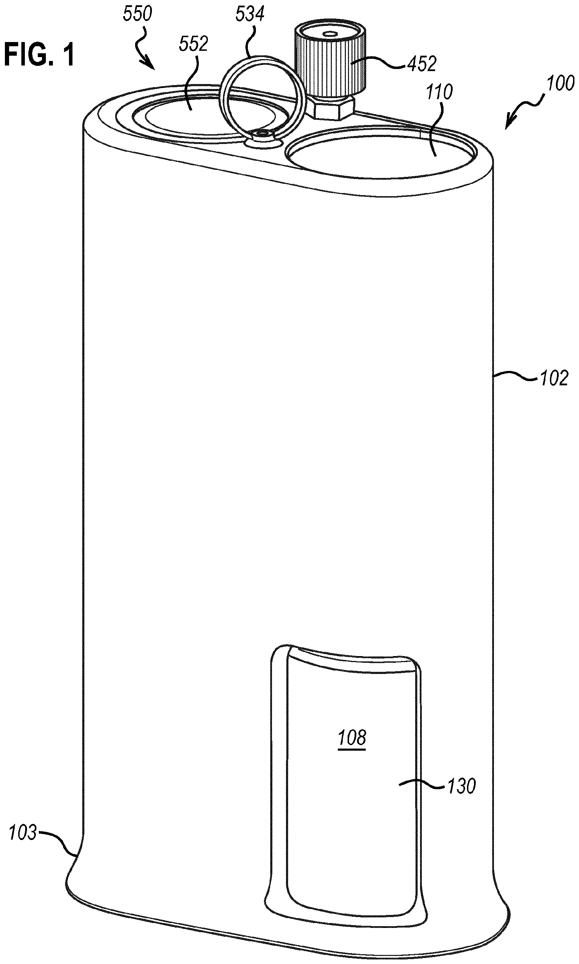

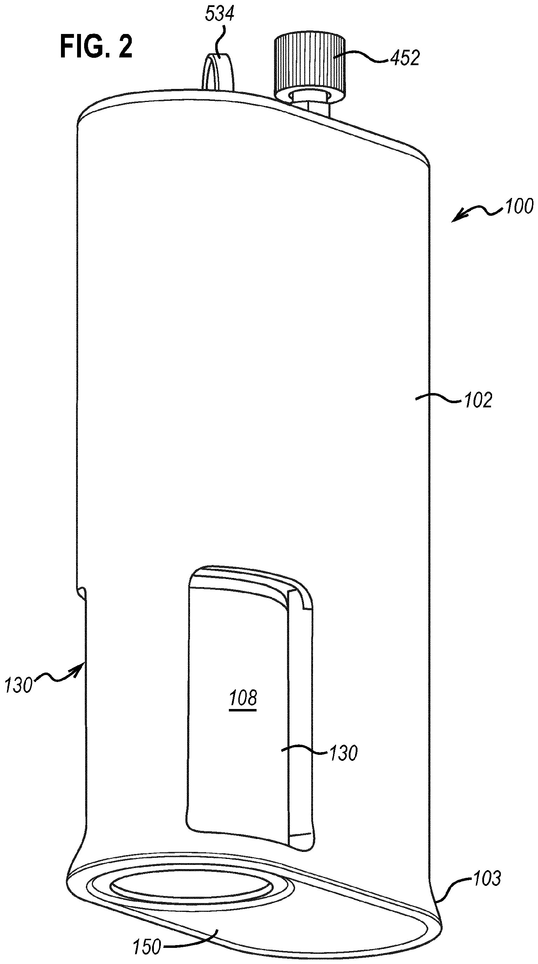

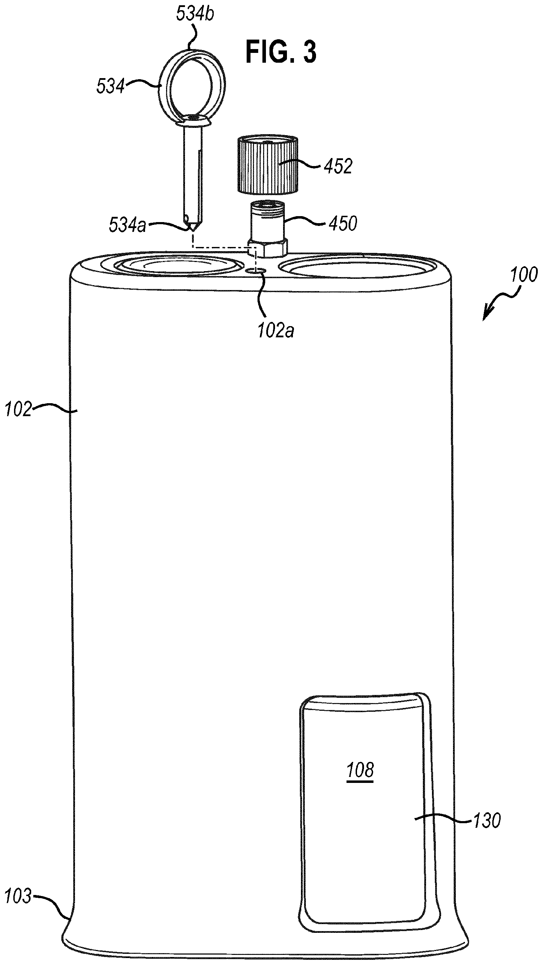

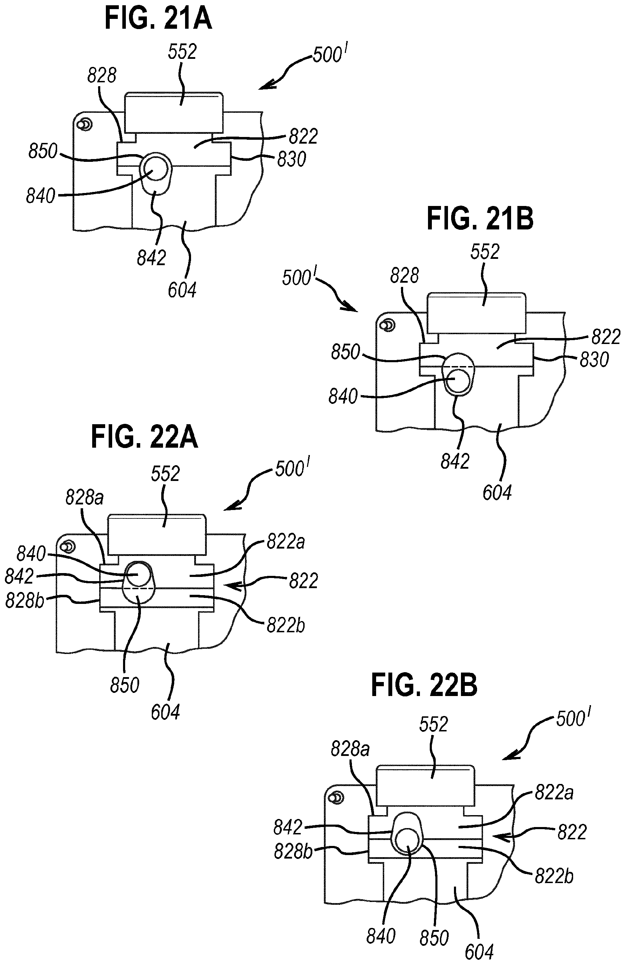

Specific reference to the features shown in each of Figures 1 to 20 should be made in order to understand the principles of the present invention as outlined below. A variant of the locking mechanism, able to be integrated into the embodiment of Figures 1 to 20, is shown in Figures 21A, 21B, 22A and 22B.

### Common features and definitions

As used herein the term "drug mixing device" means a device specifically adapted for the mixing of two or more components of a drug, for example a device which enables the transfer of a first component of a drug from a first location to a second location where mixing with a second component takes place to form the mixed drug.

A "container" is a part able to function as a temporary or permanent receptacle for holding another part, for example a "first container" for holding a first component of a drug to be mixed. The ordinal in "first container" and "second container" is used to distinguish two containers, but does not necessarily imply any limitation on the sequence in which the two containers are used or encountered. Similar considerations apply for containers with higher ordinal number.

By describing two parts as being "fluidly coupled" means that a structural connection between the two parts exists, permitting the transfer of fluid from the first part to the second part, via a fluid coupling. The term "fluidly coupled" does not mean that fluid transfer is actually occurring, only that a fluid pathway has been established such that fluid may flow when the device is used.

A "transfer member" is a structure able to operate as the structural connection between two fluidly coupled parts. The transfer member thereby provides a fluid pathway between the two parts.

An "exit transfer member" is a transfer member that provides a fluid pathway between a part of the device and the outside of the device.

A "driving fluid transfer member" is a transfer member that provides a fluid pathway for a driving fluid.

By "first component of a drug to be mixed" and "second component of a drug to be mixed" is meant a constituent part of a drug and when the constituent parts are mixed, the drug forms a drug administrable to a human or animal. The ordinal is used to distinguish the two components but is otherwise non-limiting and does not refer to a specific order except where context implies. Either component may be in a solid or fluid phase without restriction, unless the context requires otherwise. The components may further be liquid, gel, suspension or another phase. Examples include a liquid component being mixed with a solid component, or a liquid component mixed with a further liquid component. Either component may also comprise a drug in its own right, prior to mixing.

By the term "hydraulic resistance" is meant the resistance to the flow which occurs as a result of the structure through which a fluid is flowing. For example, hydraulic resistance occurs through changes or shape or direction of a tube/pipe. Hydraulic resistance is subdivided into "frictional" hydraulic resistance that arises due to momentum transfer between the fluid and the solid walls of the structure, and "local" hydraulic resistance that arises due to changes in direction of flow or configuration that results in the formation of vortices, cavitation and secondary flows, which may dissipate the fluid's mechanical energy.

By stating that two parts are "unreactive" is meant that substantially no chemical reaction occurs between the two substances when the two substances encounter each other. An unreactive substance may be chemically inert. Alternatively, it may be that two unreactive substances have little tendency to react due to their chemical properties or due to the conditions (e.g. thermal) in which the two unreactive substances encounter each other.

By describing an action as "automatic" is meant that the action occurs and may be completed without further manual intervention. The action may be initiated by manual intervention and then proceed automatically. Further, a first action may be initiated, proceed automatically and by virtue of the first action proceeding partway or to completion, an automatic initiation of a second action may also occur and by this mechanism, the second action is ultimately initiated by initiation of the first action.

By "aperture" is meant a hole or space in a part, through another part may pass or be dispensed. An aperture may be of any shape or size, and the direction in which the aperture points may be defined by a vector normal to the plane of the aperture.

By "antifoam" is meant a chemical additive or agent that reduces or hinders the formation of, or the further formation of, a foam in a process involving liquids. An alternative term for antifoam is "defoamer".

When a first part is said to be "above" a second part, the centroid of the first part is positioned above the centroid of the second part with respect to the ground. Similarly when the second part is said to be "below" the first part, the centroid of the second part is positioned below the centroid of the second part with respect to the ground.

A "specific/specified orientation" is an orientation of an object that is selected by the designer of the object to achieve a particular disposition of the object. For example, a specified orientation of an object may position a first constituent part of the object above a second constituent part of the object, relative to the ground.

The "boundary" of a part is used to describe the outermost peripheral contour of the part. The outermost periphery is not limited solely to the physical structure of the part. For example, if the part includes a port or gap, the boundary of that part includes any chords across the port or gap.

The "base" of a part is defined as the portion of the part upon which the part stands upright when left at rest on a surface such as the ground, a workbench, a table etc.. The reaction contact force due to the surface acts through the base of the part. The base may be a single substantially planar surface that comprises part of the boundary of the part, but may also be an undulating surface or more complex surface where only portions of the base and boundary are in direct contact with the surface of the ground, the workbench or the table etc..

An "opposing" relationship between two parts as used herein refers to the disposition of the two parts, which are arranged and oriented in complementary fashion about a specific location. For example, a pair of containers are in an opposing relationship if the opening of each container is oriented to point towards the opening of the other container. A pair of needles are in an opposing relationship if the needles point away from the same point in antiparallel directions.

As used herein, "shaking" of a part refers to the periodic or non-periodic agitation/stirring of a part by manual or automatic means in order to encourage movement of the part. When the part is a component of a drug to be mixed, the shaking creates larger interaction surfaces for the component, thereby aiding rapid completion of the drug mixing process.

The drug to be mixed is made of at least two components, a first component of a drug to be mixed 1000 and a second component of a drug to be mixed 1010. The process of mixing the drug may be to reconstitute the drug prior to administration to the patient. The components may be of the drug Remicade (RTM) and may be sterilised water and powdered Remicade (RTM). Nevertheless, the components may be for a different drug without affecting the operation of the drug mixing device.

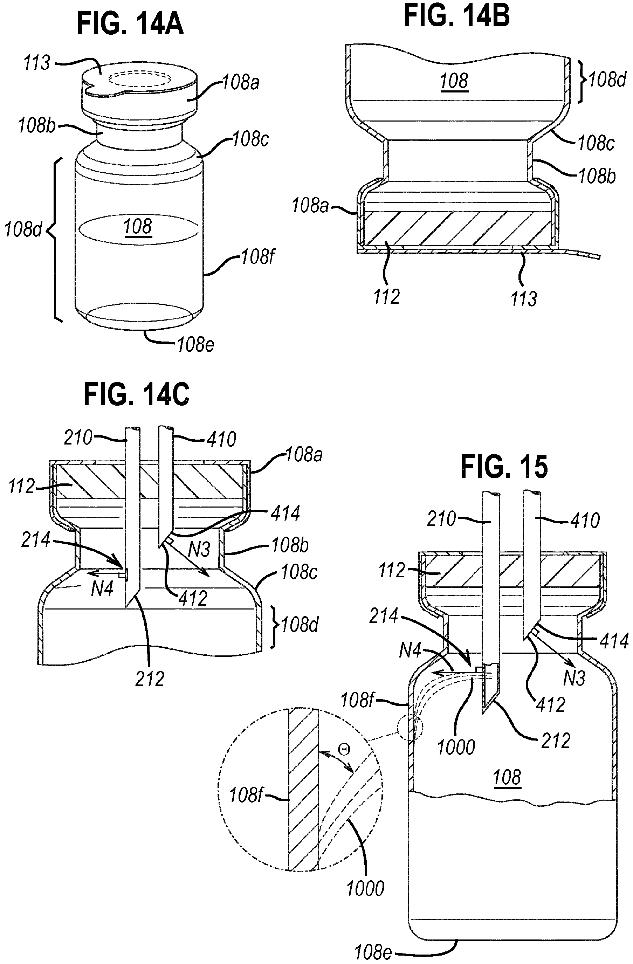

In any of the following embodiments, a container is used for each of the first and second containers by which a first drug component from the first container is mixed with a second drug component within the second container. The one or more (e.g. first and second) containers are for holding components of the drug to be mixed, and may be jars, ampules, vials, cylinders, packets or bottles. In the specific embodiment, vials 108 and 110 (the former being shown in Figure 14A) will be used as examples of containers, but wherever vial is used, this should be understood to be interchangeable with any other suitable container. Each of the exemplary vials has a fixed capacity/volume.

The maximum internal volume of each of the (e.g. first and second) containers may lie in the range from 1ml to 1000ml, and more specifically in the region of 1ml to 100ml. In a specific embodiment, the volume of each of the container's lies in the region of 1ml to 30ml. The container's internal volume may be fixed.

The one or more containers may contain an external scale indicating the volume or capacity of the container, which can be read by a user to indicate the progress of filling or emptying the container whilst the container is positioned for use in the drug mixing device.

The containers will typically be sterile, and contain an opening, closed with a closure. The closure may be one or more septa, as in the case of the vials, but alternative closures that are not septa may be used.

The containers may also include temporary protective seals, such as a plastic cover or a foil 113, to ensure the surface of the closure remains sterile prior to use.

It is understood that any containers may be sold separately from the drug mixing device, but that a drug mixing device may also be sold with the containers as a kit.

The containers may be configured to be detachably received in the drug mixing device. With the exception of the containers, the drug mixing device of the following embodiments is in an assembled state 'out of the packet' and requires no further user assembly to use, with the exception of the insertion of the containers.

Drug mixing devices according to the present invention have a range of sizes, governed principally by the yield of mixed drug that is required for administration. The yield of mixed drug determines the size of the containers of the drug mixing device, thereby determining the size of the housing, outer housing and inner support. Additionally the volume of driving fluid required to mix the drug is similarly influenced by the required yield of mixed drug.

In various examples, the drug mixing device has a height, width and length each falling in the range of 10mm to 300mm, and a first container volume, second container volume and driving fluid reservoir volume each falling in the range of 1ml to 1000ml. The specific dimensions of the drug mixing device 100 are outlined below.

| **Parameter** | **Size** |
|---|---|
| Height | 122mm |
| Width | 70mm |
| Depth | 33mm |
| First container capacity | 15ml |
| Second container capacity | 25ml |
| Driving fluid reservoir capacity | 15ml |

Though the capacity of the first container, second container and driving fluid reservoir is as above in the embodiment, each need not be full to capacity with the first component, the second component or the driving fluid respectively. For example, in a specific embodiment, the first container having capacity 15ml contains 10ml of sterilised water. The second container having capacity 25ml contains approximately 11ml of mixed drug after mixing. Similarly, the driving fluid reservoir capacity is 15ml but the driving fluid transferred is 12.9ml.

### Housing and Structure

According to an embodiment of the present invention, drug mixing device 100 includes a generally cuboidal housing 101, the housing 101 including an outer housing 102 and inner support 150 as generally shown in Figures 1 to 4. Outer housing 102 provides a protective casing for the remaining parts of the drug mixing device 100.

As can be seen from Figures 1 to 3, outer housing 102 includes a flanged base 103. The flanged base 103 has several advantages. Firstly, the flanged base 103 assists in the stability of the outer housing 102, thereby the drug mixing device 100 when the device is stood upright on a surface (such as a workbench) during use or during storage. Secondly, the flanged base 103 provides an indication to the user as to the 'right way up' of the device, since the flanged base 103 is positioned on the outer housing 102 such that the drug mixing device is intended to be stood upright on the flanged base. Outer housing 102 is a unitary moulded plastic piece configured to slot over the inner support 150 and may in addition be secured via glue or screw once slotted over the inner support 150. Outer housing 102 also defines part of the boundary 140 (see Figure 5) of the drug mixing device 100.

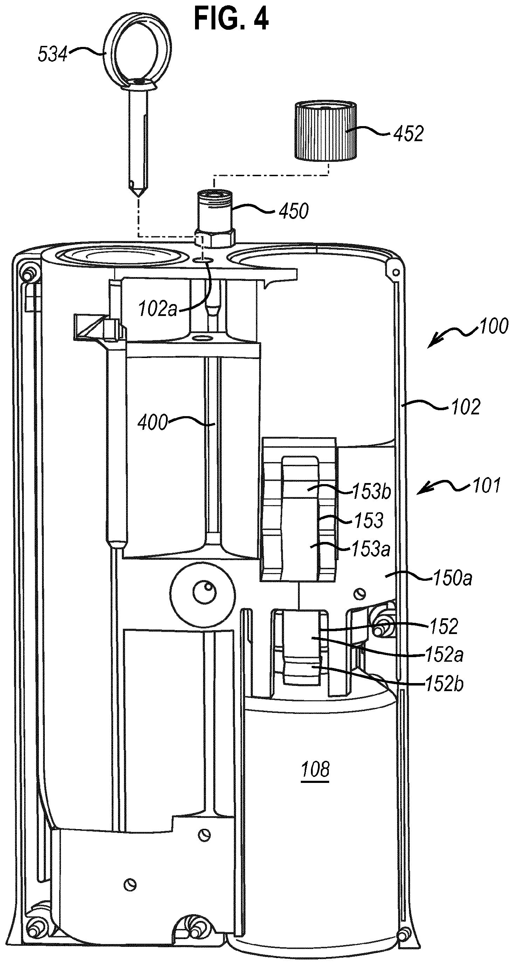

Inner support 150, as shown in Figures 4, 5 and 6, provides a supporting structure for the remaining components of the drug mixing device 100, such as the transfer member 200, the driving fluid transfer member 300 and the exit transfer member 400. Inner support 150 is moulded in two pieces 150a, 150b by conventional techniques. Once moulding is complete, the actuator 500, fluid driver 600, energy store 700 and the transfer members 200, 300, 400 are slotted into one piece 150a and the second piece 150b is then secured over the first piece 150a using a screw and nut arrangement. Alternative means of securing the two pieces 150a, 150b of the inner support 150 together, such as glue or plastic cement, or snap-fit, may be used.

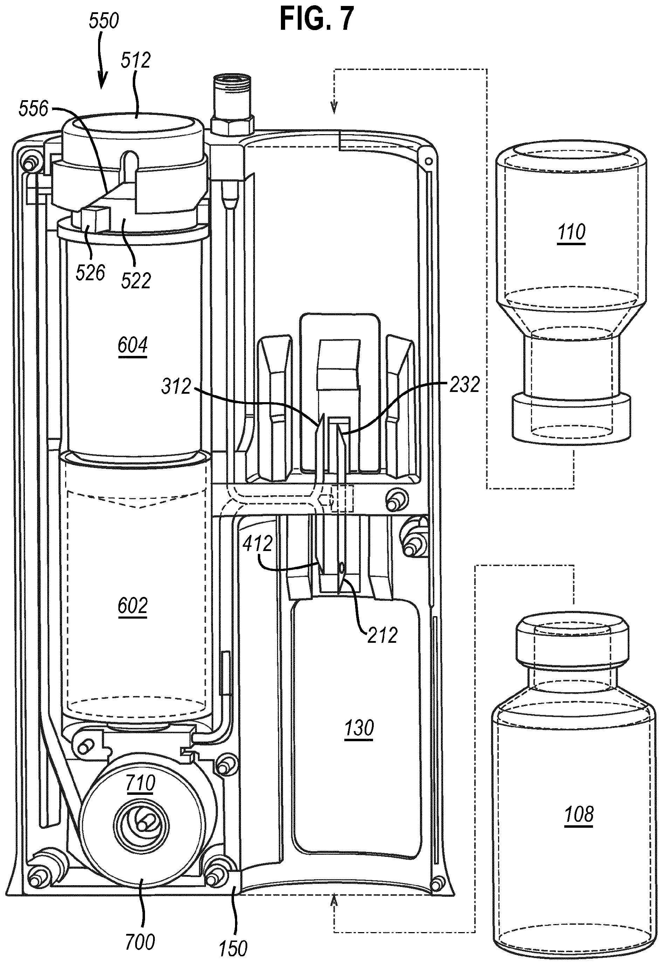

As can be seen in Figure 5, housing 101 includes a circular first port 104 and circular second port 106, each of which is dimensioned, shaped and configured to detachably receive a container such as a vial 108 (as indicated by Figure 7).

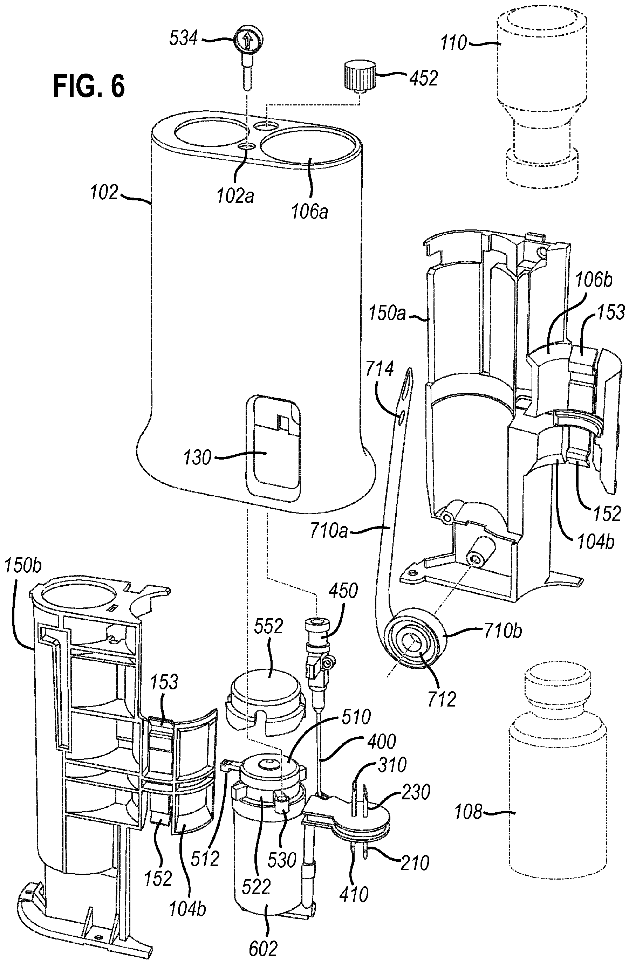

Circular first and second ports 104 and 106 each include initial apertures 104a and 106a formed in opposing ends of the outer housing 102, and guiding portions 104b and 106b formed as part of the outer surface of inner support 150 and/or the inner surface of outer housing 102. Each of the ports 104 and 106 (that is, the surfaces, the guiding portions 104b, 106b, the apertures 104a, 106a, the snap-fit members 152, 153 etc.) is moulded into the combination of outer housing 102 and inner support 150 (the latter two collectively making up the housing 101 of the drug mixing device 100). As can be seen from the combination of Figures 5 and 6, ports 104 and 106 in the specific embodiment are formed from the combination of the outer housing 102 and the inner support 150 in housing 101. Ports 104, 106, apertures 104a, 106a and guiding portions 104, 106b are chosen to be substantially or completely circular for ease of manufacturing.

The specific embodiment users two cylindrical vials 108 and 110 as exemplary containers. Cylindrical vial 108 includes a top 108a, a neck section 108b, a tapered shoulder section 108c and a main body 108d as shown in Figures 14A and 14B. Cylindrical vial 110 also includes a circular top 110a, a neck section 110b, a tapered shoulder section 110c and a cylindrical main body 110d. Top 108a contains an opening that is closed by septum 112 that is configured to seal vial 108 in the absence of the penetration of the septum 112 by a needle. A similar opening, with closure by a septum 114 is contained in top 110a (not shown in Figure 14A or 14B, but of similar construction to that of vial 108). Vials 108 and 110 are manufactured from substantially transparent glass, but may be made from plastic or alternative material. Furthermore, the closure of one or more of top 108a and 110a need not be a septum.

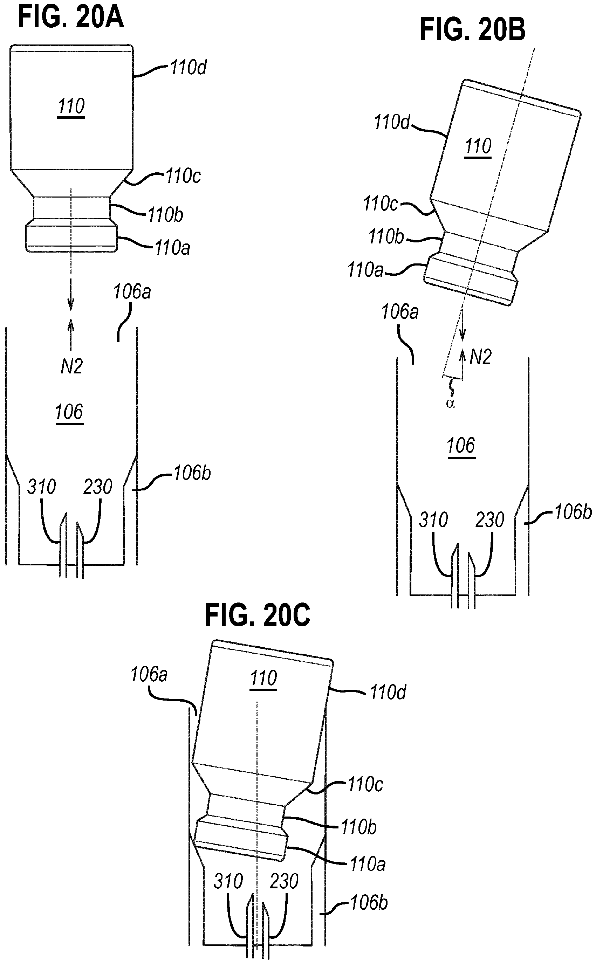

In the present example, cylindrical main body 108d of vial 108 has a diameter that matches the diameter of circular first port 104 and cylindrical main body 110d of vial 110 has a diameter that matches the diameter of circular second port 106. Aperture 104a defines a direction normal N1 to the plane of the aperture 104a. Aperture 106a defines a direction normal N2 to the plane of the aperture 106a. In the present example, N1 and N2 are antiparallel to each other, though this configuration is not required. An example of aperture 106a is shown in Figures 20A and 20B.

As shown generally in Figures 7 and 8, a consequence of the different sizes of circular ports 104 and 106, the vial 108 cannot be pushed into circular port 106 through aperture 106a because during the insertion, one or more of the top 108a, neck 108b, shoulder section 108c or main body 108d will have a diameter that exceeds the diameter of aperture 106a. Thus vial 108 is unable to be received in port 106. Avoiding incorrect insertion of the vial 108 into port 106 is advantageous in the drug mixing device where a unidirectional mixing process is required because the correct location and sequence of the mixing of components of the drug to be mixed is vital for creating an effective mixed drug.

The configuration of the first port 104 is such that top 108a, neck 108b, shoulder section 108c and main body 108d may each pass through aperture 104a of port 104. The top 108a, neck 108b, shoulder section 108c may each pass through the port 104 in a direction parallel to normal N1, or alternatively, may pass through the port at an angle α oblique to normal N1. A similar configuration for second port 106 exists for insertion of vial 110. Aperture 106a of port 106 with normal N2 may also receive vial 110 either parallel to normal N2, or at an oblique angle α. Both of these options are illustrated for port 106 in Figures 20A to 20C.

An oblique angle α of insertion represents a minor error on the part of the user during use because the vial 108 is designed to be received in a specific orientation where the axis of the vial is parallel or antiparallel to the normal N1. In the instance where the angle α is oblique, upon passing through aperture 104a, as the vial continues to be pushed in to the housing, top 108a encounters guiding portion 104b. Guiding portion 104b has a tapered configuration which cams the top 108a and thereby the vial 108 into the specific orientation as the vial 108 continues to be inserted. Thus the guiding portion 104b reorients the vial 108 during its insertion into the port, thereby adjusting the position and alignment of the vial 108. The constituent aperture 106a and guiding portion 106b of port 106 have a similar action on vial 110 if vial 110 is inserted into the second port 106 at an oblique angle α, as shown in Figures 20A to 20C.

As a result of the guiding portions 104b and 106b, the user may speedily insert vials 108, 110 into ports 104, 106 through the apertures 104a, 106a, without concern for the vials' precise alignment with respect to directions N1 and N2 respectively, relying on the guiding portions 104b and 106b to ensure that the alignment of the vials is correct by the time insertion is complete. Guiding portions 104b and 106b also ensure that, upon full insertion of the vials 108 and 110 respectively, the vials are unable to translate in directions perpendicular to directions N1 and N2 respectively.

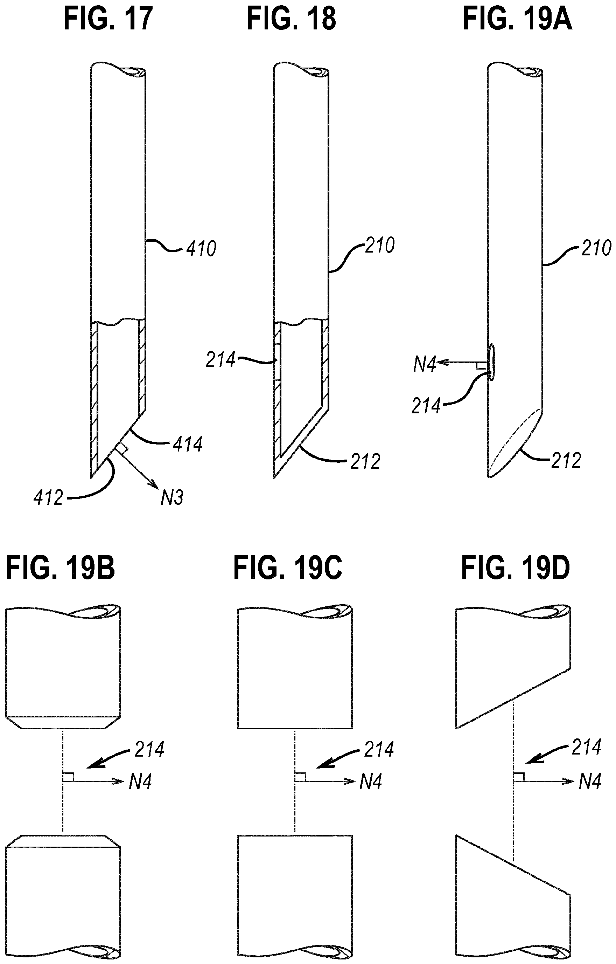

When the vial 108 is pushed further into port 104 by the user, the top 108a of the vial first encounters the tip 212 of needle 210 of transfer member 200, the transfer member 200 being supported on inner support 150 of drug mixing device 100. Continued pushing of vial 108 into port 104 results in penetration of septum 112 of vial 108 because septum 112 is pierced by the needle tip 212. Tip 212 has a slanted profile that arrives at a point to aid penetration and to avoid needle coring of septum 112 as shown in Figure 17. The configuration once the vial 108 is fully inserted is shown in Figure 14C.

Penetration/piercing of the septum 112 by needle 210 achieves several effects. Firstly, the penetration permits transfer of substances into and out of the vial 108 through the transfer member 200. The interior of transfer member 200 is therefore fluidly coupled to the vial 108. Secondly, the penetration attaches the vial 108 to inner support 150, with the needle 210 of the transfer member 200 assisting in prohibiting movement of the vial 108 in directions perpendicular to the direction N1.

Further continued pushing of vial 108 into results in penetration of septum 112 by tip 412 of needle 410 because the tip 412 pierces the septum 112. Needle 410 is a portion of exit transfer member 400, the exit transfer member 400 being supported on inner support 150 of drug mixing device 100, as shown in Figures 5 and 6.

In the exemplary embodiment needles 210 and 410 extend from the same surface of the inner support 150. During insertion of the vial 108 into port 104, tip 212 of needle 210 is always encountered by the septum 112 of vial 108, prior to tip 412 of needle 410 encountering the septum 112 of vial 108, because the extension of needles 210 and 410 is dissimilar; needle 210 extends further away from the surface of support 150 than needle 410.

Penetration/piercing of the septum 112 by needle 410 also achieves several effects. Firstly, the penetration permits transfer of substances into and out of the vial 108 through the exit transfer member 400. The interior of exit transfer member 400 is therefore fluidly coupled to the vial 108. Secondly, the penetration attaches the vial 108 to inner support 150, with the needle 410 of the transfer member 400 further assisting in prohibiting movement of the vial 108 in directions perpendicular to the direction N1. Thirdly, the combination of needle 210 and needle 410 and the closure of the vial 108 restrict any clockwise or anticlockwise rotation of vial 108 about an axis parallel to the direction N1.

During insertion of the vial 108 into port 104, the neck 108b of vial 108 is also secured in position by a snap-fit member 152. Snap-fit member 152 has an arm 152a and a tooth 152b, the arm 152a extending substantially parallel to the direction of insertion of vial 108 and to the direction N1 (see Figure 6). Tooth 152b is disposed on the distal end of the arm and is configured to engage with neck 108b of vial in order to prevent movement of the vial 108 parallel or anti-parallel to the direction N1. By this means of attachment to the inner support 150, movement of the vial once inserted is restricted.

When the vial 110 is pushed into port 106 by the user, the top 110a of the vial first encounters the tip 312 of needle 310 of driving fluid transfer member 300, the driving fluid transfer member 300 being supported on inner support 150 of drug mixing device 100. Continued pushing of vial 110 into port 106 results in penetration of septum 114 of vial 110 because septum 114 is pierced by the needle tip 312. Tip 312 has a slanted profile that arrives a point to aid penetration and to avoid needle coring of septum 114.

Penetration/piercing of the septum 114 by needle 310 achieves several effects. Firstly, the penetration permits transfer of substances into (and out of) the vial 110 through the driving fluid transfer member 300. The interior of driving fluid transfer member 300 is therefore fluidly coupled to the vial 110. Secondly, the penetration attaches the vial 110 to inner support 150, with the needle 310 of the driving fluid transfer member 300 assisting in prohibiting movement of the vial 110 in directions perpendicular to the direction N2.

Further continued pushing of vial 110 into results in penetration of septum 114 by tip 232 of needle 230 because the tip 232 pierces the septum 114. Needle 230 is another portion of transfer member 200.

In the exemplary embodiment needles 310 and 230 extend from the same surface of the inner support 150, as shown in Figure 6. During insertion of the vial 110 into port 106, tip 312 of needle 310 is always encountered by the septum 114 of vial 110, prior to tip 232 of needle 230 encountering the septum 114 of vial 110, because the extension of needles 310 and 230 is dissimilar; needle 310 extends further away from the surface of support 150 than needle 230.

Penetration/piercing of the septum 114 by needle 230 also achieves several effects. Firstly, the penetration permits transfer of substances into and out of the vial 110 through the transfer member 200. The interior of transfer member 200 is therefore fluidly coupled to the vial 110. Secondly, the penetration attaches the vial 110 to inner support 150, with the needle 230 of the transfer member 200 further assisting in prohibiting movement of the vial 110 in directions perpendicular to the direction N2. Thirdly, the combination of needle 310 and needle 230 and the closure of the vial 110 restrict any clockwise or anticlockwise rotation of vial 110 about an axis parallel to the direction N2.

During insertion of the vial 110 into port 106, the neck 110b of vial 110 is also secured in position by a snap-fit member 153 in a similar fashion to snap-fit member 152. Snap-fit member 153 has an arm 153a and a tooth 153b, the arm 153a extending substantially parallel to the direction of insertion of vial 110 and to the direction N2 (see Figures 5 and 6).. Tooth 153b is disposed on the distal end of the arm 153a and is configured to engage with neck 110b of vial in order to prevent movement of the vial 110 parallel or anti-parallel to the direction N2. By this means of attachment to the inner support 150, movement of the vial once inserted is restricted.

In each case, restriction of the movement of vials 108 and 110 enables the most effective seal between the vials and the needles to be provided.

In each case, when vial 108 is fully inserted into port 104 and vial 110 is fully inserted into port 106, the base of the body portion of each vial 108d, 110d lies within the boundary 140 of the outer housing 102 (as shown by Figure 8). By avoiding a portion of the vial 108 protruding beyond the boundary of the outer housing 102, there is no likelihood that the vial 108 will be knocked sideways whilst it sits in port 104 and thus no sideways knock would cause a dislodging lever force at the septum 112, risking compromising the seal around needles 210 and 410, or accidental removal of the vial. Furthermore, avoiding a protruding vial means that the vial 108 does not limit whether or not the drug mixing device 100 may be stood on that surface. For similar reasons, vial 110 is fully inserted into port 106 and does not protrude beyond the boundary 140 of the outer housing 102 to achieve the same advantages.

Full insertion of vial 108 into port 104 and vial 110 into port 106 establishes a fluid coupling not only between the transfer member 200 and the vials, but also between vial 108 and vial 110, via the transfer member 200. Thus a fluid pathway between vial 108 and vial 110 is established. Similarly, full insertion of vial 108 establishes a fluid coupling between vial 108 and the exit transfer member 400, and thereby a fluid coupling and a potential fluid pathway between vial 108 and the outside world. Likewise, full insertion of vial 110 establishes a fluid coupling between driving fluid transfer member 300 and vial 110, thereby a fluid coupling and a potential fluid pathway between vial 110 and the means for driving the driving fluid.

Full insertion of the vials results in needle 210 of transfer member 200 extending through septum 112 and into vial 108 further than needle 410 of exit transfer member 400. Similarly, needle 310 of driving fluid transfer member 200 extends through septum 114 and into vial 110 further than needle 230 of transfer member 200. The extension of the needles provides a relative difference between the locations of the needle's apertures.

As is shown in Figures 1 to 3, outer housing 102 contains windows 130 offering views into ports 104 and 106. In the embodiment, the windows are simply gaps in the surface of the outer housing 102. The window 130 permits a user to visualise directly whether or not a vial 108, 110 is present/absent in the first port 104 or the second port 106. Since the vial 108 has substantially translucent or transparent character, both the window 130 and the vial 108 permit direct visualisation of the component of the drug to be mixed.

As can be seen in Figures 6, 7 and 8, in the embodiment, inner support 150 supports needles 210, 230, 310 and 410 in a manner such that needles 210 and 410 point in the direction N1, whereas needles 230 and 310 point in the direction N2 and the directions N1 and N2 are antiparallel to each other. Upon full insertion, needles 210 and 410 pierce septum 112 and needles 230 and 310 pierce septum 114. Thus vials 108 and 110 are located in an opposing relationship (see Figure 8), whereby the openings and septa on each container point towards each other. An opposing relationship in combination with the drug mixing device 100 standing upright on its flanged base 103 has the advantage of signalling to the user the correct way up of each of vials 108 and 110 for use in the device 100, aiding quick insertion of the vials 108, 110. Additionally, the opposing relationship affords the drug mixing device 100 the opportunity to have a short transfer member 200, and affords the opportunity for mixing to be assisted by gravity.

As is seen generally in Figures 4 to 8, alongside ports 104 and 106 that may receive vials 108 and 110, inner support 150 also includes fluid driver 600. Fluid driver 600 is fluidly coupled to driving fluid transfer member 300. In addition, when vial 110 is inserted into port 106, fluid driver 600 is fluidly coupled to vial 110 via driving fluid transfer member 300. When vials 108 and 110 are fully inserted into drug mixing device 100, as a consequence of the arrangement of transfer members and containers, the fluid driver 600, the driving fluid transfer member 300, vial 110, transfer member 200, vial 108, and exit transfer member 400 are fluidly coupled. The transfer members 200, 300, 400 may contain valves to control the direction of the flow along the fluid pathway.

Within inner support 150, fluid driver 600 is aligned with ports 104, 106 and dimensioned such that the fluid driver 600 occupies a space adjacent to the ports 104, 106 in the inner support 150, within the two pieces of inner support 150a, 150b. This configuration is compact, leading to an overall size of the drug mixing device 100 that has little or no unused or redundant space. The fluid driver is fluidly coupled to the driving fluid transfer member 300 and thereby to needle 310.

Within inner support 150 resides actuator 500, positioned at one end of the fluid driver 600. Actuator 500 is interfaced with both energy store 700 and fluid driver 600 and occupies a further portion of the inner support pieces 150a, 150b. The configuration is again compact, minimizing the space used by the actuator 500 within drug mixing device 100. Actuator 500 is actuatable by a user from outside the outer housing 102 using a trigger 550. In the specific embodiment, actuator 500 interfaces mechanically with the trigger 550, the trigger comprising a depressible button 552 that protrudes through part of the outer housing 102.

Also within inner support 150 is energy store 700. Energy store 700 is configured to occupy space adjacent to the ports 104, 106 and below fluid driver 600, and to be mechanically connected to fluid driver 600 and to actuator 500. Energy store 700 provides a source of stored energy which can be converted into work in order to drive a drug mixing operation upon actuation of the mixing operation by actuator 500. Energy store 700 in the specific embodiment is a flat spring, which interfaces with the fluid driver 600.

Whilst the preceding describes the specific embodiment of the invention shown in Figures 1 to 20, alternative embodiments of the housing and structure of the drug mixing device 100 exist without departing from the scope of the present invention.

In alternative embodiments, the inner support 150 and outer housing 102 are made by additive manufacturing processes, such as 3D printing. Furthermore, the inner support 150 may comprise more than two pieces, as may the outer housing 102. Either or both of the inner support 150 and outer housing 102 may be made by injection moulding.

In alternative embodiments, outer housing 102 may feature rings, protrusions, indentations or other topology on the outer surface in order to aid gripping of the device by the user.

In alternative embodiments, the ports 104 and 106 may take different shapes, for example, either port may be hexagonal, or octagonal. Furthermore, though both ports are circular, there is no requirement for the ports 104 and 106 to have the same shape and port 104 might be square, whereas port 106 might be triangular. In this instance, the incorrect container entering the port may be avoided due to the incorrect shape, in addition or instead of the incorrect size and may result in both ports being unable to receive the incorrect container. Additionally, the structure of the ports 104, 106 may instead be provided solely by the outer housing or the inner support.

In alternative embodiments, the positioning and alignment adjustments of the vials may be achieved by a different guiding portion. For example a threaded portion might be used, whereby the vial is screwed into the guiding portion. A threaded portion would provide the additional benefit of being a further means of restricting the movement of the vial during the attachment and guiding process and once the vial is attached.

In alternative embodiments, the order of insertion of the vials 108, 110 may also be prescribed. A protective member, such as a plastic membrane, may occlude one or other of the ports. The membrane may include an indication of the order of insertion of the vials (for example, with labelling "Insert this vial side first" or similar) to encourage the user in the correct order of insertion. Alternatively still, the protective member may include a mechanism whereby the insertion is prevented if the order of insertion of the vials is incorrect. For example, the member may occlude the port, the aperture or a portion thereof, and the occlusion not released until the first vial in the prescribed order has been inserted. By such a mechanism, the user is forced into using the correct order of insertion. The use of such a member may also simultaneously provide the advantage of ensuring that the needles remain sterile.

In alternative embodiments, more than one snap-fit members 152, 153 to assist in restricting the movement of vials 108 and 110 respectively. For example, two snap-fit members may be provided on opposing sides of a port so that each engages the neck of the vial. Additionally, there is no requirement for both vials 108 and 110 to have the same number of snap-fit members.

In alternative embodiments, the one or more windows 130 may contain a substantially transparent or translucent sheet of plastic, glass or other suitable material. Any such window may still permit the component of the drug to be mixed to be visualised by the user. In further alternative embodiments, portions of the outer housing 102 may be removed in order to expose the component of the drug to be mixed.

More than two containers may be provided, for example three containers, whereby three components of the drug to be mixed must be kept separate before mixing. In this embodiment the drug mixing device includes an additional port for the extra container, and additional needles. A manifold, with a series containers that positioned in corresponding ports is possible.

### Push and Forget

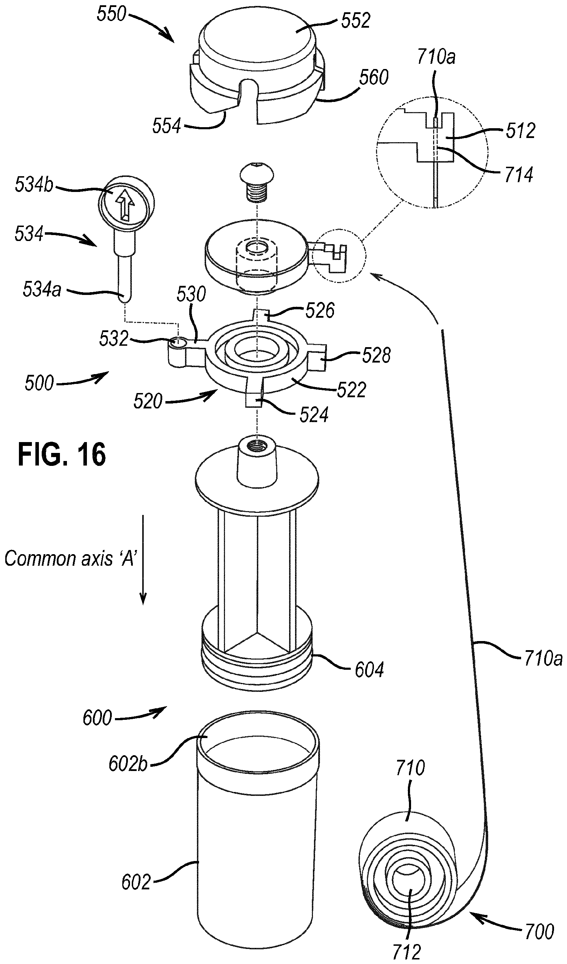

According to an embodiment of the present invention and as referenced to above, drug mixing device 100 includes an actuator 500, shown generally in Figures 5 and 16. Actuator 500 is configured to respond to trigger 550, and provided the actuator 500 is not in a locked state, actuator 500 interfaces with fluid driver 600 to cause mixing of the drug. Actuator 500 hence couples the trigger 550 to the fluid driver 600, as can be seen from Figure 5.

In the specific embodiment and as shown in Figure 16, trigger 550 is a depressible circular button 552. The depressible button 552 protrudes through the outer casing 102 of the housing 101, and includes a concave contour adapted to receive a user's digit. Additionally, the depressible button 552 includes a distinguishing indicia which enables its rapid identification by an inexperienced user. In the specific embodiment, the indicia is a green or 'go' button.

Actuator 500 includes plate 510, hook 512 and a locking mechanism 520. Locking mechanism 520 operates in two states: a locked state, where the trigger 550 is prevented for causing the actuator 500 to commence mixing, and an unlocked state wherein the actuation of the trigger 550 by the user results in the mixing of the drug. In the specific embodiment, the locking mechanism 520 interfaces with the button 552 to prevent movement of actuator 500 and thereby prevent actuation of fluid driver 600. The structure of actuator 500, plate 510, hook 512 and locking mechanism 520 is outlined in the following paragraphs.

Locking mechanism 520 includes a substantially circular ring 522 disposed in slot around a portion of the actuator 500. Ring 522 includes protrusions 524, 526, and 528, alongside arm 530, which each extend radially outwards from diametrically opposed locations on the ring in a 'cross' configuration. Arm 530 with hole 532 is disposed diametrically opposite protrusion 528. Hole 532 is configured to receive the distal end of pin 534, as shown in Figure 8.

As is also shown in Figure 16, the underside of button 552 includes four camming surfaces 554, 556, 558 and 560. Each camming surface is configured to interface with one of protrusions 524, 526, 528 or arm 530. Each of the camming surfaces is configured to turn the translational depressive movement of the button 552 into a rotational movement of the circular ring 522.

Camming surfaces 554 and 556 interface with protrusions 524 and 526 of locking mechanism 520 of the actuator 500, as shown in Figure 5. Each of protrusions 524 and 526 initially resides in 'L'-shaped slots 162, 164, one slot on each piece of inner support 150a, 150b ('L' shaped slots can be seen in Figure 6 on pieces 150a and 150b). Camming surfaces 558 and 560 interface with protrusion 528 and arm 530. Protrusion 528 and arm 530 also initially reside in 'L'- shaped slots formed when inner supports 150a and 150b are placed together. The 'L' shaped slots subdivide into two portions: a first portion before the corner of the 'L' shape and a second portion after the corner of the 'L' shape (see again Figure 6).

In the first position engagement between the first portion of the slots and the protrusions/arm prevents ring 522 from moving in the direction of a common axis 'A' (see Figure 16), thereby preventing actuator 500 from actuating. In the second position, movement of the protrusions 524, 526, 528 and arm 530 in a direction along the common axis 'A' is possible because the protrusions 524, 526, 528 and arm 530 may move along the second portion of the 'L' shaped slot, the second portion of the 'L' shaped slot extending in the direction of the common axis 'A'. Rotary movement of protrusions/arm along the first portions of their respective slots to the corner of the 'L' moves the protrusions/arm from the first position to the second position. This rotary movement moves the protrusions from a first position where actuation of the actuator 500 is prohibited, to a second position where actuation of the actuator 500 is permitted. Ring 522 thus acts as a latch, preventing actuation of actuator 500 in its first position and permitting actuation of actuator 500 in the second position. Furthermore, once, protrusions 524, 526, 528 and arm 530 are in the second position and are able to move along the second portion of the 'L' shaped slot, protrusions 524, 526, 528 and 530 act as guides in the slots on pieces 150a and 150b of the inner support 150, guiding the movement of the actuator down the common axis 'A'.

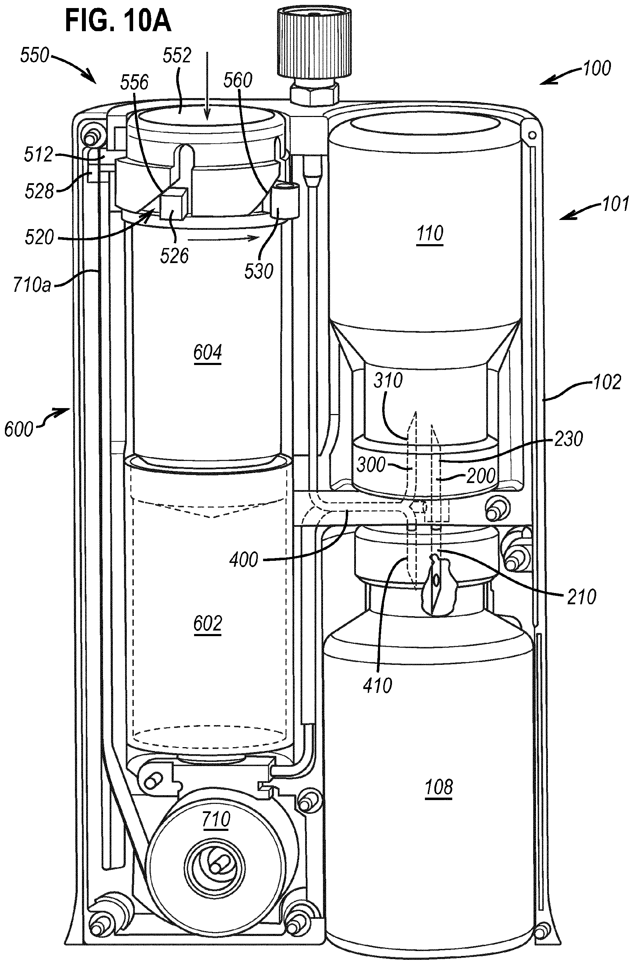

The protrusions 524, 526, 528 and arm 530 are configured to move along their respective first portions when cammed by camming surfaces 554, 556, 558 and 560 to the second position referred to above, as shown in Figure 10A. However, protrusions 524, 526, 528 and arm 530 are only free to move within their respective first portions if the locking mechanism 520 is in an unlocked state. Movement of protrusions 524, 526, 528 and arm 530 is prevented in the locked state because ring 522 is unable to rotate.

In the locked state of locking mechanism 520, pin 534 extends from hole 532 through the housing 101 and through pinhole 102a in the outer housing 102 to the outside of the outer housing 102. Pin 534 is an elongated member with a tapered distal end 534a to enable easy alignment with hole 532 and a handle 534b to assist in removal. The handle 532b prevents the pin 534 from falling into housing 101 because it will not pass through pinhole 102a in the outer casing 102. A user must withdraw pin 534 from hole 532 by gripping and pulling the handle 534b in order to remove the pin 534 from hole 532, and thereby enable rotation of ring 522. If the pin 534 has not been removed, rotation of the ring is prevented. If the ring 522 cannot be rotated, protrusions 524, 526, 528 and arm 530 cannot move in their respective slots and so camming surfaces 554, 556, 558 and 560 cannot move either. As a result of this mechanism, pin 534 acts as a key in locking mechanism 520, that when locked, prevents depression of button 552 in trigger 550.

Even with pin 534 removed, camming of the protrusions 524, 526 and 528 and arm 530 by camming surfaces 554, 556, 558 and 560 occurs only when button 552 is depressed. Removal of pin 534 unlocks the locking mechanism 520, leaving the mechanism in an unlocked state. As a result, removal of pin 534 does not immediately cause the commencement of the mixing process, and the pin 534 may be replaced (re-locked), for example, if mixing is to be postponed.

With pin 534 removed, depressing the button 552 cams protrusions 524, 526, 528 and arm 530, rotating locking ring 522 from the first position to the second position. Upon the ring 522 reaching second position, actuator 500 may then immediately move along the common axis 'A' and interface with piston 604 of fluid driver 600 to commence mixing of the drug. In this respect, movement of the trigger 550 causes actuator 500 to commence mixing of the drug.

As is shown in Figure 16, actuator 500 includes plate 510 and hook 512. Plate 510 is axially aligned with ring 522 of locking mechanism 520 about the common axis 'A', and plate 510 cannot move along the common axis 'A' unless the ring 522 also moves along the common axis 'A' too. Phrased another way, ring 522 prevents movement of plate 510 unless ring 522 is able to move along the common axis 'A' and ring 522 may only move along the common axis 'A' when the ring has been cammed to the second position by button 552.

Hook 512 is connected to energy store 700. Hook 512 forms the connection by which stored energy causes mechanical movement of the actuator 500, and thereby actuation of the fluid driver 600.

Initially, hook 512 sits above the corner of the 'L' shaped slot associated with protrusion 528. Hook 512 is aligned with and would be able to move in the direction of the common axis 'A' along the second portion of the 'L' shaped slot associated with protrusion 528, but for the fact that such movement is prevented by the ring 522 unless ring 522 has reached the second position. As a result, stored energy from energy store 700 may not do work to cause movement of actuator 500 to commence mixing of the drug.

In the unlocked state and once each protrusion 524, 526, 528 and arm 530 has been cammed to the second position at the corner of its 'L' shaped slot, each is then free to move along the second portion of the slot which is oriented in the direction along the common axis 'A'. In the second position, the interface between the protrusions/arm and the first portion of the slot that prevented movement of the ring 522 along the common axis 'A' has been removed. Hence, protrusions/arm and ring 522 may move in a direction along the common axis 'A' and ring 522 no longer blocks plate 510, which may also move in a direction along the common axis. Hence, stored energy from energy store 700 may be released to do work to cause movement of actuator 500 to commence mixing of the drug.

The movement of protrusion 528 to the second position by cam 558 causes protrusion 528 to become aligned with hook 512. Since protrusion 528 can, in the second position, move along the second portion of the slot in the direction of the common axis 'A', hook 512 may also move along the common axis 'A'. Thus both hook 512 and protrusion 528 advance along the second portion of the 'L' shaped slot during the mixing process, as energy store 700 does work on the actuator 500.

Hook 512 is adapted to be reinforced by protrusion 528 when the two components are aligned in the second position. Since energy store 700 acts only on one side of the actuator 500, the energy store is prone to causing rotation about an axis along the centre of the actuator 500 (an axis parallel to the protrusions 524 and 526), This rotation may cause a skewed movement of actuator 500. Skewed movement is avoided by having hook 512 mechanically reinforced by the protrusion 528 to avoid such a rotation. Similar mechanisms may be employed to avoid deleterious effects of alternative connections between the energy store and the actuator.

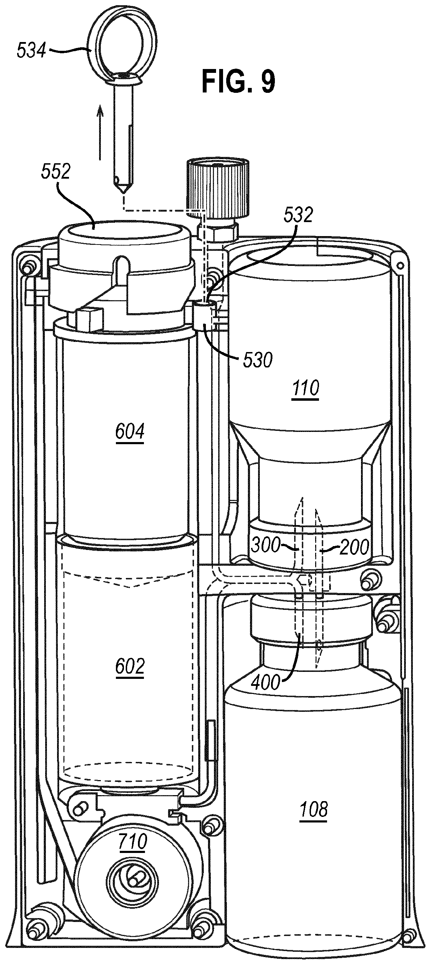

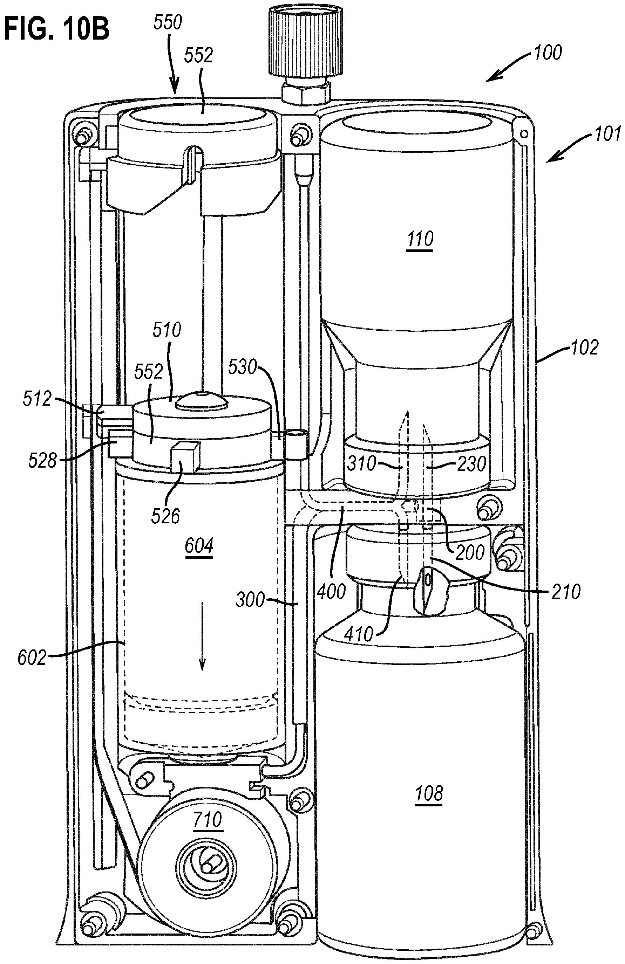

By the above described mechanism, once the pin 534 has been removed (see Figure 9) and button 552 pressed (Figure 10A), the actuation of actuator 500, thereby the actuation of fluid driver 600 occurs automatically without further user interaction (Figure 10B). Significantly, this means that the mixing of the first component of the drug to be mixed 1000 with the second component of the drug to be mixed 1010 occurs in a substantially reproducible fashion, and a user is not required to move an actuator manually to mix the drug. Automatic mixing improves the reliability of the mixing of the two components because the rate of mixing is set by the nature of the components of the drug mixing device 100 (type of energy store 700 etc.) and not by any manual action of the user. The mixing will also be completed to the same extent in each drug mixing device 100. This is particularly effective if the user of the device has limited dexterity, or is performing other actions whilst the mixing process proceeds.

Furthermore, whilst it is envisaged that this device will mainly be used by a healthcare practitioner in a medical practice, the reproducibility of the mixing means that a non-healthcare practitioner may use the device and produce the mixed drug in identical fashion to a healthcare practitioner. The drug mixing device 100 may also be used by a patient themselves, which may be necessary in emergency situations.

Whilst complete mixing of the drug is designed to take place automatically (once triggered) without further patient manual interaction from the user, the user may nevertheless shake the drug mixing device 100 to promote mixing during the mixing process.

Whilst the preceding describes the specific embodiment of the invention shown in Figures 1 to 20, alternative embodiments of the push and forget mechanism of the drug mixing device 100 exist without departing from the scope of the present invention.

In alternative embodiments, the trigger is not a depressible button. For example, the trigger may instead be a switch or a rotation knob. Equally the button may not be depressible, but could rather be a pullable feature, whereby pulling the feature triggers the actuator 500.

In alternative embodiments, a different locking mechanism may be used. For example, an electronic locking mechanism, a magnetic locking mechanism or a different kind of mechanical locking mechanism. One specific alternative mechanical locking mechanism, described in detail below and shown in Figure 21 is a gravitational locking mechanism.

Furthermore, the positioning of the locking mechanism to block the movement of the actuator may be varied without compromising the operation of the present invention. For example, the locking mechanism may be a means that prevents a user from interacting with the trigger, such as a cover or a lock external to the outer housing 102 of the drug mixing device 100 that stops the user interacting with the button 552.

In alternative embodiments, there may be more or fewer camming surfaces on the underside of the button, and the direction of the camming action (clockwise or anticlockwise) may be the same or may differ for each cam. Furthermore, the locations of the cams may differ about the ring 522. A single cam may also interact with multiple protrusions on the ring sequentially, in order to provide a 'staged' unlocking mechanism, where each protrusion is cammed in sequence..

In further alternative embodiments, the locking mechanism may not be aligned about a common axis with piston 604. For example, a hydraulic system may operate between actuator 500 and piston 604 with the locking mechanism located in a tubing between the two. This tubing may permit a side-side-by-side orientation of the actuator 500 and piston 604.

Alternatively or additionally, further fail-safe mechanisms and locking mechanisms may be present, whereby each locking mechanism or fail-safe mechanism must be in the unlocked or open state in order to trigger the actuator to commence automatic mixing of the drug.

In addition to the push and forget mechanism, the drug mixing device may contain a visual or auditory signal that indicates to the user that mixing is complete. For example, the piston 604 may 'click' when the piston has completed the automated mixing process. Alternative signals would also be possible, and may be provided by mechanical, electronic or magnetic means.

### Pressure Driven Mixing

According to an embodiment of the present invention and as referred to above, drug mixing device 100, once vials 108 and 110 are fully inserted, establishes a fluid coupling between each of fluid driver 600, driving fluid transfer member 300, vial 110, transfer member 200, vial 108 and exit transfer member 400, in the arrangement shown in Figure 8. Each of these fluidly coupled structures forms a portion of a fluid pathway for at least one fluid in the drug mixing device 100.

Drug delivery device 100 further includes energy store 700 alongside fluid driver 600. During use that has been initiated by actuator 500, the stored energy is released to do work on one or more fluids, to thereby facilitate mixing of the drug. Work is done on one or more of the fluids as a result of actuation of actuator 500, by a further actuator. In the specific embodiment, the further actuator is the fluid driver 600.

Fluid driver 600 includes a cylindrical driving fluid container, referred to as a reservoir 602 herein, and a piston 604. Prior to actuation of the fluid driver 600, reservoir 602 is filled or partially filled with driving fluid. When the reservoir is filled with driving fluid, pressure transmission through the driving fluid is almost instantaneous (depending on the driving fluid) because driving fluid forms an essentially uniform medium inside the reservoir 602, leading to a quick response of the driving fluid to being driven by the fluid driver 600. A cylindrical reservoir is used for ease of manufacture.

In the present embodiment, reservoir 602 is pre-filled with a specified quantity of driving fluid. The driving fluid is unreactive with the first component of the drug to be mixed. In the embodiment the driving fluid is air because of its low cost, though other unreactive fluids may be used. The volume of reservoir 602 is fixed and lies in the range 1ml to 20ml and the quantity of driving fluid also lies in the range 1 ml to 20ml. In the specific embodiment, the reservoir 602 has a volume of 15ml and is able to transfer 12.9ml of driving fluid to the first container.

With further reference to Figure 8, reservoir 602 includes an exit aperture 602a, fluidly coupled to fluid transfer member 300 (the other end of driving fluid transfer member 300 being needle 310, extending into vial 110). Reservoir 602 also includes an entrance aperture 602b. Piston 604 is cylindrical and is dimensioned and configured to fit snugly within the entrance aperture 602b to provide a leak-free interface between reservoir 602 and piston 604 to prevent the driving fluid escaping from the reservoir through entrance aperture 602b. Piston 604 is also configured to move within the volume of reservoir 602. One end of cylindrical piston 604 thereby occludes entrance aperture 602b because initially (and prior to the release of any stored energy) piston 604 is at rest at or just inside entrance aperture 602b. The extent to which the piston 604 sits inside the entrance aperture 602b is governed by the need to ensure the above snug-fit and a leak free interface. The snug-fit between entrance aperture 602b and piston 604 is achieved by their both having a closely matching circular cross-section, such that no driving fluid leaks through aperture 602b, nor that any other fluid is able to enter the driving fluid reservoir 602.

During the mixing of the drug, energy is released from energy store 700, in order to do work on piston 604, which moves into stationary reservoir 602. Movement of piston 604 into stationary reservoir 602 reduces the volume available in reservoir 602 that is available for driving fluid, thereby increasing the pressure within the reservoir and causing the expulsion of driving fluid from reservoir 602 through exit aperture 602a and into driving fluid transfer member 300. Movement of the piston 604 thereby does work on the driving fluid, eventually arriving at the configuration of Figure 10B. Whilst it is relative movement of the piston 604 and reservoir 602 that reduces the volume, movement of the piston 604 into a stationary reservoir is used in order to allow a stationary fluid coupling between reservoir 602 and driving fluid transfer member 300. Movement of the piston 604 coincides with movement of actuator 500, including its locking mechanism 520 (protrusions 524 and 526 slide down the slots in pieces 150a and 150b, one of which can be seen in Figure 10B).

With vial 110 fully inserted into port 106, driving fluid transfer member 300 fluidly couples reservoir 602 to needle 310, establishing a fluid pathway between fluid driver 600 and vial 110. Movement of driving fluid from reservoir 602 through driving fluid transfer member 300 causes the eventual expulsion of the driving fluid into vial 110 from needle 310. In the specific embodiment where the driving fluid is air, expulsion of air through needle 310 into vial 110 causes bubbles.

When the drug mixing device 100 is stood upright on a surface on its flanged base 103, the air bubbles rise upwards, away from needle 310 because the bubbles are more buoyant than the first component of the drug to be mixed 1000. This leads to an accumulation of air at the top of vial 110, whilst the needle 310 and needle 230 each remain submerged in the first component of the drug to be mixed 1000.

The accumulation of driving fluid (air) in vial 110 leads to an increased pressure on the first component of a drug to be mixed 1000 because a decreased volume is available for the first component of the drug to be mixed in vial 110. As a consequence of the increased pressure and reduced volume, work is done on the first component of the drug to be mixed 1000. The first component of the drug to be mixed 1000 enters transfer member 200 via needle 230. Needle 230 is configured to be as low within the vial 110 as possible in order to minimise the residual amount of first component of the drug to be mixed 1000 left in the vial 110.

The pressure driven flow of driving fluid into the vial 110 prevents the first component passing back into needle 310, but a precautionary one-way valve preventing any of the first component of the drug to be mixed 1000 from passing back into needle 310 could also be included.

With both vials fully inserted into ports 104 and 106, transfer member 200 fluidly connects the first vial 110 to the second vial 108 and establishes the fluid pathway between the two vials. The fluid pathway enables the first component of the drug to be mixed that enters the transfer member 200 as a result of the above process to flow to the second vial 108. Flow between the two vials is pressure driven, but, with the drug mixing device 100 stood upright on a surface on its flanged base 103, is also assisted by gravity. Transfer member 200 may include a one-way valve to facilitate unidirectional flow of the first component of the drug to be mixed 1000.

The first component of the drug to be mixed 1000 flows through the transfer member 200 and is dispensed into second vial 108 from needle 210. The second vial 108 contains a second component of a drug to be mixed 1010, alongside a volume of air. The dispensing of the first component from needle 210 causes both first and second components of the drug to be mixed 1000, 1010 to be present in the same container and thereby to mix.

Dispensing of the first component of the drug to be mixed 1000 into vial 108 results in a reduced volume being available for second component of the drug to be mixed 1010 and the air originally in the vial 108. Consequently, as the first component of the drug to be mixed enters the vial 108 there is an increase in pressure in the vial 108, because the volume of the vial 108 is fixed. In order to alleviate any build-up in pressure, vial 108 is fluidly connected to exit transfer member 400 via needle 410. At the other end of exit transfer member 410 is a connector 450, covered by a vent cap 452. The vent connector is disposed on the outer housing 102 of the drug mixing device 100. The vent connector 452 permits the release of air from within the exit transfer member 400 to the outside via a one-way valve. Exit transfer member 400 therefore establishes a fluid pathway by which the air in vial 108 may be released.

The above described mechanism results in the dispensing of driving fluid from the fluid driver 600 into the driving fluid transfer member 300 and then through needle 310 into vial 110. By the above mechanism, the first component of the drug to be mixed 1000 then flows from vial 110, into transfer member 200, as a result of work being done on the first component by the driving fluid. Also by the above mechanism, the first component of the drug to be mixed is relocated through transfer member 200 into vial 108 and thereby the first and second components of the drug to be mixed 1000, 1010, are mixed.

Whilst the preceding describes the specific embodiment of the invention shown in Figures 1 to 20, alternative embodiments of the pressure driven mixing that occurs in the drug mixing device 100 exist without departing from the scope of the present invention.

It is not necessary for the reservoir to have a fixed volume, provided that work may be effectively transferred from the energy store 700 to the fluid driver 600 and to the driving fluid upon actuation by the actuator 500. For example, the reservoir may be a flexible bag and the volume available for the driving fluid within the bag is reduced by actuation of the fluid driver 600.

Similarly, it is not necessary for the containers to have a fixed volume, provided that work may be effectively transferred from the energy store 700 via the fluid driver 600 to the first component of the drug to be mixed 1000 upon actuation by the actuator 500. For example, the first container may be a flexible bag and the volume available for the first component 1000 within the bag is reduced by actuation of the fluid driver 600

In alternative embodiments, the driving fluid may also be both unreactive and inert. Alternatively still, the driving fluid may be reactive with the first component of a drug to be mixed, but separated therefrom by a barrier within the container which prevents mixing of the reactive driving fluid and the first component of the drug to be mixed. The barrier may be a flexible, non-porous membrane disposed in the container 110.

In alternative embodiments, different mechanism for dispensing driving fluid from the fluid driver 600 are used. For example, a different geometry of piston, entrance aperture and reservoir may be used, such as geometries with a square or elliptical cross-section. Alternatively, whilst the piston and entrance aperture share the same cross section, they may have a different cross section to the reservoir, in either dimension or shape and so 'slack' volume in the driving fluid reservoir may be useful.

In alternative pressure drive embodiments, a threshold pressure may need to be reached before the driving fluid is dispensed from the reservoir 602. Use of threshold afford control over the timing of mixing, since no mixing occurs before driving fluid is dispensed into the driving fluid transfer member.

In further embodiments, the rate of dispensing of the driving fluid from reservoir 602 is controlled, for example by varying the dimensions of the exit aperture 602a. A smaller aperture increases the rate of flow of driving fluid for the same movement of the piston 604. Additionally or alternatively, the rate of dispensing may be controlled by varying the rate of movement of piston 604.

In alternative embodiments, a different actuator might be used to move driving fluid out of the reservoir, for example a pump, such as a peristaltic pump, an osmotic pump or a mechanical or electrical pump. Alternatively still the reservoir may be a flexible membrane that may be impinged upon by an actuator.

In further alternative embodiments, unintended leakage of driving fluid may be prevented by common methods, such as disposing rubber O-ring or similar between the mobile piston 604 and the stationary reservoir 602 to effect the leak-free interface between these two parts.

In alternative embodiments, the reduction in volume in the reservoir 602 may arise from movement of the reservoir (and the fluid coupling to the driving fluid transfer member) relative to a stationary piston, or a combination of movements of both piston and reservoir.

In alternative embodiments, the driving fluid reservoir may not be pre-filled and instead maybe fillable or refillable via a sealable port. The fluid driver may then be reused for multiple drug mixing operations.

In further alternative embodiments, the driving fluid transfer member 300 may be primed with driving fluid, and thereby may store a volume of driving fluid in addition to reservoir 602. If so, movement of piston 604 into reservoir 602 will result in driving fluid being dispensed from needle 310 almost immediately because of the continuum of driving fluid in the reservoir and driving fluid transfer member. A continuum such as this means that the driving fluid is dispensed from needle 310 more rapidly upon actuation of the fluid driver.

In alternative embodiments, the amount of first component of the drug to be mixed that is transferred to the second vial may be calibrated. Calibration may be by partial actuation of the fluid driver to limit the amount of driving fluid to be only part of the fluid stored in the reservoir. Further alternatively, the amount of first component of the drug to be mixed that is transferred to the second vial may be calibrated by changing the extension of needle 230. When the drug mixing device 100 is in use in the upright position stood on its flanged base 101, the needle 230 extends into vial 110. Increasing or reducing the amount that needle 230 extends into vial 110 will increases or reduces the residual first component left in the vial during the mixing process, affording the user greater control over the ratios of first and second component to be mixed.

In further alternative embodiments of the pressure driven mixing, the energy store 700 that comprises one of a compressed spring or a compressed gas, whereby the compression is released in order that the spring or gas may do work on the fluid driver 600 in order to cause mixing of the first component of the drug to be mixed 1000 with the second component of the drug to be mixed 1010.

### Drawdown Mechanism

As described above in the specific embodiment, the drug delivery device 100 includes energy store 700 that provides the source of energy which does work upon the actuator 500 in order to mix first component of the drug to be mixed 1000 with the second component of the drug to be mixed 1010 to form the mixed drug 1020.

In the specific embodiment, the energy store 700 is an elastic member 710 that is coupled to the actuator 500 by hook 512, as shown in Figures 5, 6 and 16. Elastic member 710 is a spool-mounted constant force flat metallic spring including a substantially flat spring arm 710a and a roll 710b. The spring arm 710a refers to the extended portion of the spring, which contains a hole 714 for hook 512 at its distal end. Hole 714 for receiving hook 512 provides a stable interface between hook 512 and arm 710a, without the need for adhesive (an adhesive might disintegrate over time). The roll 710b refers to the portion mounted on spool 712. During the release of energy from elastic member 710, the length of the arm 710a shortens as the arm is wound around the roll/spool. A spool mount 712 is used to avoid the friction that would be caused by a cavity mounting.

Elastic member 710 is positioned in inner support 150 between the two pieces 150a, 150b, with arm 710a extended along the edge of the fluid driver 600, comprising reservoir 602 and piston 604. When the device is stood upright on the flanged based 103, spool 712 and roll 710b are positioned below the reservoir 602 and hence when extended arm 710a is retracted, the piston 604 is drawn down into the reservoir 602 through entrance aperture 602b. Positioning the spool of the flat spring beneath the reservoir 602 means that there is no requirement in housing 101, or within drug mixing device 100 to provide space for a bottomed-out elastic member 710 storing energy above actuator 500 (which would be released in order to push the actuator 500 into piston 604).

In addition to the above, flat arm 710a is aligned with and the flat surface of arm 710a substantially conforms to the external contour of the piston 604 (see the position of arm 710a in Figure 5), the reservoir 602 and the actuator 500, minimising the space and footprint required in this portion of the housing 101 required to accommodate the elastic member 710 and more generally, the energy store 700..

Elastic member 710 which is initially attached to hook 512 in a tensioned (extended) state. In this tensioned state, the spring stores elastic potential energy that may be converted into work. The release of the stored elastic potential energy in elastic member 710 to move actuator 500 is prevented by ring 522 with protrusions 524, 526, 528 and arm 530 that collectively prevent movement of the ring 522 due to their location in their respective 'L' shaped slots of the inner support 150. In the locked state, since the ring 522 cannot move, plate 510 and hook 512 cannot move and thus arm 710a is unable to retract from its initial extension. Thus elastic member 710 is initially held in a tensioned state by the combination of ring 522, plate 510 and hook 512.

Upon releasing of the locked state to the unlocked state elastic member 710 still cannot move, until trigger 550 has caused protrusions 524, 526, 528 and arm 530 to move from their first position, through the first portion of their 'L' shaped slots to the second position. Since, in the second position movement of actuator 500 along the common axis 'A' is no longer prevented, elastic member may be released. Elastic member 710, previously held in a tensioned (extended) state, is able to release the tension by retracting arm 710a in a direction towards spool 712, transitioning the arm 710a progressively to the roll 710b the spool 712 and roll 710b, eventually to arrive in a substantially non-extended state. In doing so, hook 512, attached to plate 510 of actuator 500, and is drawn downwards as arm 710a retracts. In tandem, ring 522, protrusions 524, 526, 528 and arm 530 move downwards as arm 710a retracts. The movement of actuator 500 commences movement of fluid driver 600, commencing the driving of the driving fluid contained within the reservoir 602 through the exit aperture 602a and into the driving fluid transfer member 300. As explained above, the movement of this driving fluid causes the mixing of the first component of the drug to be mixed 1000 with the second component of the drug to be mixed 1010. The completed movement of piston 604 is shown in Figure 10B.

Whilst elastic member 700 provides is a constant force spring, the force provided thereby may be selected by the user in order to bring about a desired rate of mixing of the first and second components of the drug to be mixed. By selecting the force applied during the release of energy from the energy store 700, the user may calibrate the rate of mixing. When a constant force spring is used, the turbulence in the driving fluid is minimised.

By the above described mechanism in the specific embodiment, space saving is made whereby a bottomed-out elastic member need not be positioned above the actuator 500, thereby freeing-up space in the housing 101 for alternative uses by other components of the device. As a consequence, the drug mixing device 100 has fewer space requirements above the actuator 500, leaving more space for other parts of the device (e.g. the locking mechanism 520), or alternatively permitting the overall housing 101 to be smaller.

Whilst the preceding describes the specific embodiment of the invention shown in Figures 1 to 20, alternative embodiments of the drawdown mechanism in the drug mixing device 100 exist without departing from the scope of the present invention.

In alternative embodiments, the elastic member may be made of alternative materials, such as laminates or polymers, depending on the simplicity of manufacturing and usage requirements.

In alternative embodiments, a different form of elastic member may be used. For example, a coil spring may draw down the piston 604. Additional space-saving may be achieved if the coil spring is wrapped around at least a portion of the actuator that cause mixing of the drug (for example, the fluid driver 600, or a part thereof). Wrapping the coils of the spring around the fluid driver provides an additional space-saving within the housing 101, because the gap inside the coil spring is occupied by the fluid driver 600.

A non-constant force elastic member may be implemented as an alternative elastic member if a variable force is required. A variable force elastic member would afford a non-constant rate of movement of piston 604, which would cause a non-constant rate of mixing of the first component of the drug to be mixed 1000 with the second component of the drug to be mixed 1010. The non-constant force elastic member may obey Hooke's law.

A composite elastic member may be implemented, providing multiple constant force springs in tandem or back to back. The application of these elastic members may be simultaneous, or may be staged in order to adjust the rate of mixing partway through the mixing process.

The means of attaching hook 512 to arm 710a may be varied. For example, superglue may be used. Alternatively, the hook might be positioned at the distal end of arm 710a and the hole might be within part of the actuator 500.

### Drug Mixing Device and Fluid Transfer Assembly

In the embodiment of the present invention, once the first component of the drug to be mixed 1000 and the second component of the drug to be mixed 1010 have been mixed in the second vial 108, a mixed drug 1020 has been prepared in that vial of the drug mixing device 100, which is generally in the configuration shown in Figure 10B. The mixed drug 1020 must then be extracted from the drug mixing device 100 and administered to the patient at the appropriate time for treatment. The appropriate time may be immediately after mixing, or may be some interval later in the event that a particular time must lapse in order for the correct drug behaviour to occur (for example, initially the drug may not be fully prepared, but after five minutes the drug is suitable for administration).

In the specific embodiment, the drug mixing device 100 containing the mixed drug 1020 is stood upright on its flanged base 103 on a surface, such as a table or a workbench. In this configuration vent connector 450 points away from the base 103 of the drug mixing device 100. At this time, mixed drug 1020 resides in vial 108 and neither needle 210 nor needle 410 is submerged. Needle 410 extends away from the surface of the inner support 150 by less that than 11mm, which is less than the extension away from the support of needle 210 (which extends away from support 150 by 13mm). Thus needle 410 does not extend into vial 108 as much as needle 210. The needle extensions are governed in part by the thickness of the septum 112, which must be penetrated, but generally the needle extensions may fall anywhere in the range of 1mm to 30mm. A large range in needle extensions is afforded without risk of needle sticks because of the inaccessibility of the needles when outer housing 102 is positioned over inner support 150.

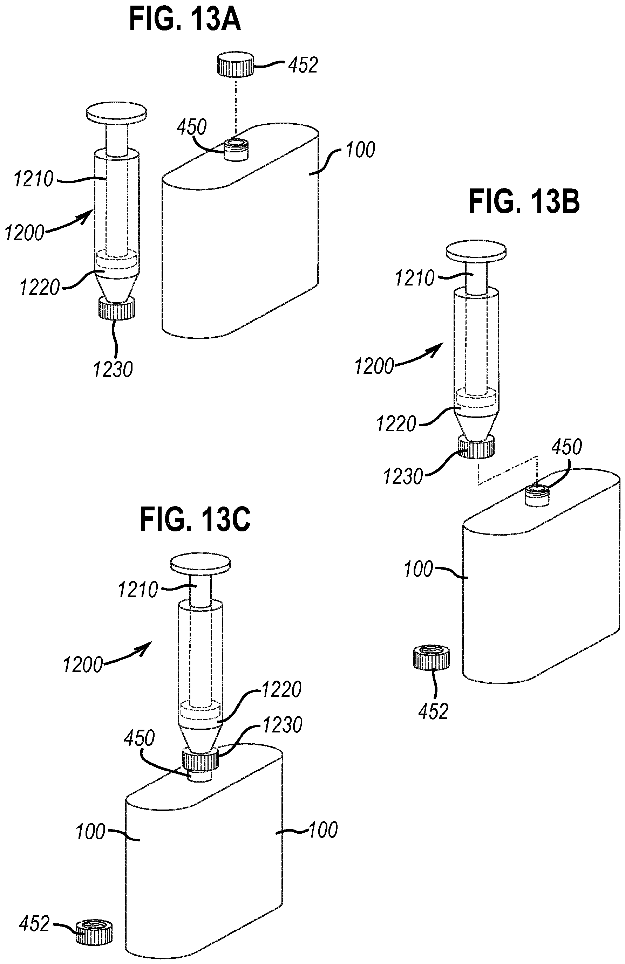

As shown in Figure 13A, the user of the device, who may be a healthcare practitioner, removes vent cap 452 from connector 450.

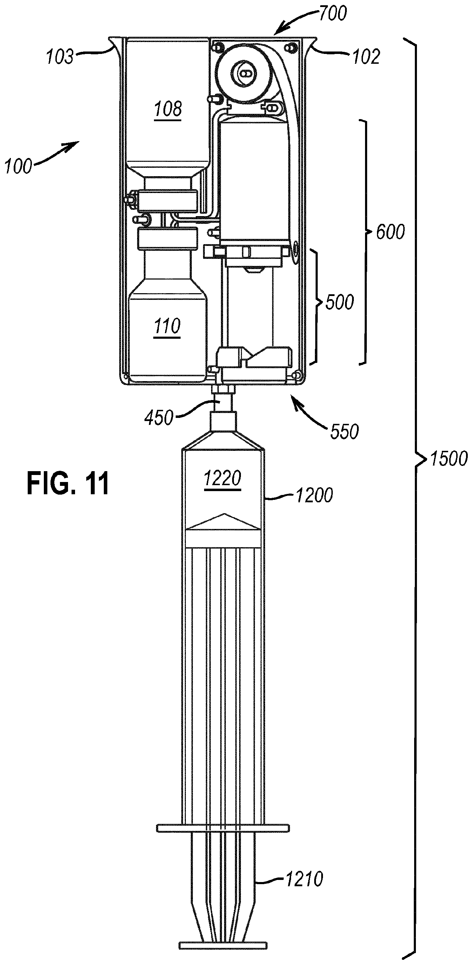

The user then takes drug administration device and forms a connection to the drug mixing device 100. In the embodiment shown in Figure 13B, the drug administration device is a syringe 1200, and connects the syringe 1200 to the connector 450 to form a fluid transfer assembly 1500. Fluid transfer assembly is therefore formed of the composite of drug mixing device 100 and syringe 1200, shown in Figures 11, 12 and 13C.

Syringe 1200 comprises a retractable syringe plunger 1210, which extends into a syringe container 1220. Initially, the syringe is empty and the plunger 1210 pushed fully into container 1220, although the syringe may contain further components for administration in alternative embodiments, provided that the plunger 1210 may retract. The capacity of the syringe lies in the region of 1 ml to 1000ml because this capacity reflects the amount of mixed drug 1020 to be administered.

The syringe 1200 has a female Luer connection 1230 on the end of container 1220 in order to provide the first portion of a leak-free connection to the drug mixing device 100. Connector 450 forms the second portion of a leak-free connection between syringe 1200 and drug mixing device 100. Connector 450 is a standard Luer connector male portion. One advantage of providing a male Luer connector on the drug mixing device 100 and the female connector on the syringe is that the connector 450 is standardised to be connected to many types of syringe 1200, which commonly employ female Luer connectors.

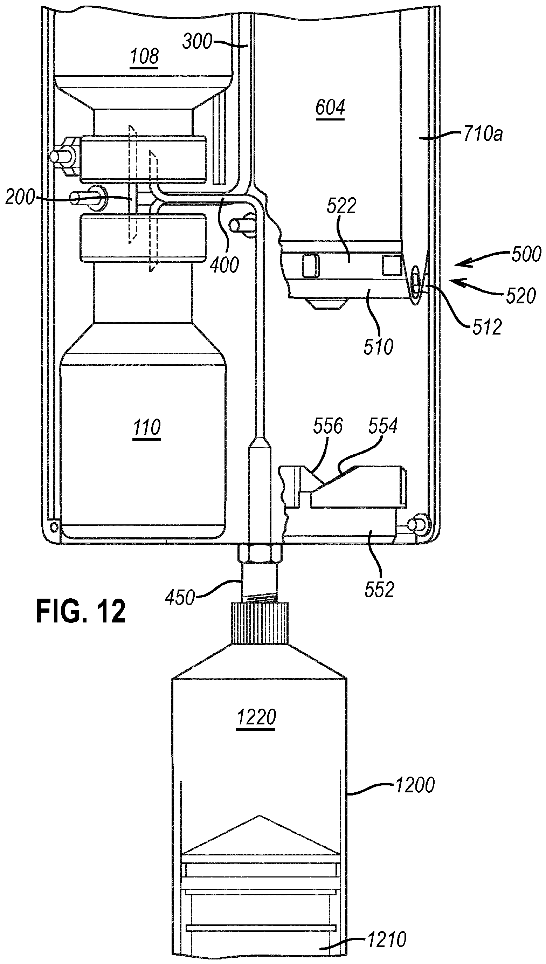

As shown by Figure 12, the connection results in a fluid coupling between exit transfer member 400 and the syringe 1200. The establishment of the fluid coupling provides a fluid pathway between the exit transfer member 400 and the syringe 1200, and since the other end of the exit transfer member is fluidly coupled to vial 108, between vial 108 and syringe 1200.

Upon securing, the flanged base 103 of the drug mixing device 100 supports the composite fluid transfer assembly 1500 with the syringe 1200 positioned above the drug mixing device 100, relative to the ground in the configuration of Figure 13C.

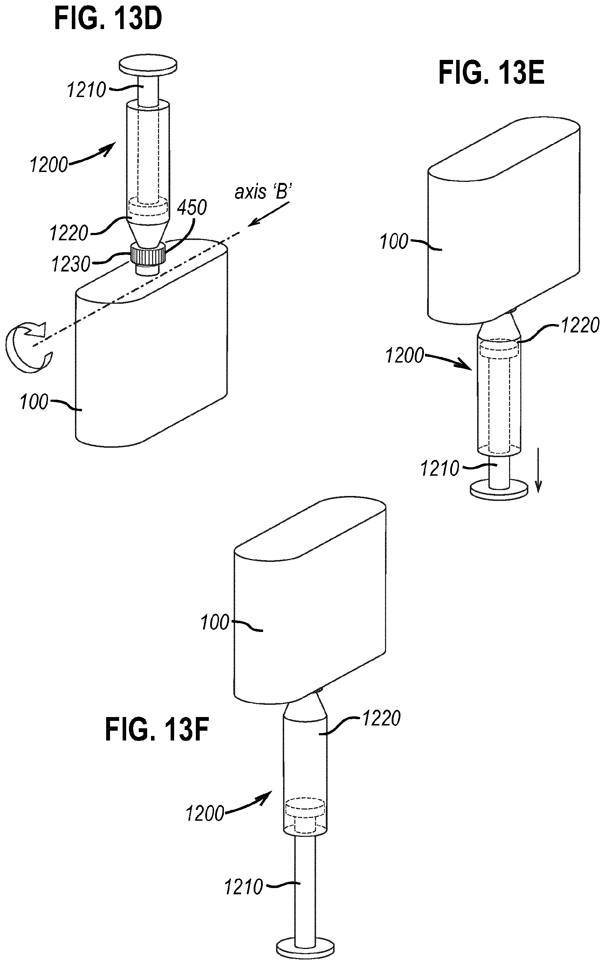

Once the assembly has been prepared, the healthcare practitioner then picks up the fluid transfer assembly and inverts the assembly by rotating the assembly by approximately 180 degrees about an axis passing through the plane of the connector 450 (for example, axis 'B' as shown in Figure 13D). In doing so, the syringe 1200 is moved to be positioned below the drug mixing device 100 and the assembly is said to be in an inverted configuration.

Whilst the inverted configuration is the specified orientation in the specific embodiment, it is noted that the present invention does not rely precisely on achieving full inversion of the fluid transfer assembly. The requirement is to move the drug mixing device 100, previously below the syringe 1200, to a position where it is above the syringe in relation to the ground.

Once the inverted configuration/specified orientation has been achieved as shown in Figure 13E, the mixed drug 1020 present in vial 108 submerges both needles 210 and 410. Needle 410 includes aperture 414, through which the mixed drug 1020 may be withdrawn into exit transfer member 400 and then into container 1220 of syringe 1200. Withdrawal occurs due to the retraction of syringe plunger 1210 by the user (see Figures 13E and 13F), which reduces the pressure inside the container 1220 in order to draw mixed drug from vial 108 to the container 1220. In the inverted configuration/specified orientation, the flow of fluid through the exit transfer member 400 to the container 1220 is also gravitationally assisted, meaning that less work must be done by the user to achieve an overall flow of fluid from vial 108 to the container 1220.

In the inverted configuration/specified orientation, the driving fluid used to drive the drug mixing process accumulates in the top of the vial 108 (the top being the opposite end to the needles 210 and 410) and thereby the vacuum-lock, which would reduce the ability to withdraw the mixed drug 1020 into the syringe 1200, is prevented. This mechanism of avoiding the vacuum lock avoids further complication in the transfer members of the device.

The advantage of the sequence of movements made with the fluid transfer assembly lies in the familiarity of these movements to healthcare practitioners. In other contexts, healthcare practitioners provide a vial with a fluid and a syringe, establish a fluid coupling between the syringe and the vial when the vial is positioned on a surface. The healthcare practitioner then inverts the assembly of the vial and syringe and draws out the fluid into the syringe. The fluid assembly of the present invention, comprising a drug mixing device and a drug administration device are used in similar fashion. The use in a similar fashion seizes upon this familiarity to reduce the likelihood of human error occurring at this stage of the drug preparation and administration process.

Whilst the preceding describes the specific embodiment of the invention shown in Figures 1 to 20, alternative embodiments of the drug mixing device and fluid transfer assembly exist without departing from the scope of the present invention.

Alternative drug administration devices may be used which are not syringes, such as patches or infusion devices. Further alternatively, a syringe with a needle attached may be used. The needle may penetrate into the drug mixing device in order to establish a fluid coupling with the drug mixing device, though this would require additional manipulation of a needled administration device. However, the exit transfer assembly 400 may be dimensioned to accommodate the needle and may contain a reinforced configuration to prevent any strain being exerted on the needle when the fluid transfer assembly is moved between orientations.

Whilst a one-to-one correspondence between the drug mixing device 100 and the syringe 1200 has been described, the exit transfer member 400 of the drug mixing device 100 may subdivide into multiple pathways arriving at multiple connectors 450, each of which may be connected to a drug administration device, such as a syringe. The fluid transfer assembly may be considered the composite of the drug mixing device 100 and multiple drug administration devices.

In alternative embodiments, the Luer connection between the syringe 1200 and the drug mixing device 100 may have an alternative arrangement, whereby the female portion is provided on the drug mixing device and the male portion on the syringe.

Alternative connectors other than Luer connectors may be used to form a fluid coupling between the drug administration device and the exit transfer member. For example, a pierceable septum may be provided instead of the connector 450 on the drug mixing device 100. The syringe may be provided with a needle and the septum pierced in order to establish a fluid coupling between the two components. The vent of the drug mixing device may be located elsewhere. Further alternatively, a stopcock may be used.

In further alternative embodiments, a different method of preventing the vacuum-lock may be used, such as an additional vent within drug mixing device 100.

The specific embodiment shows a direct connection between the drug mixing device 100 and the syringe 1200, but, whilst this is most familiar, this is not required. A tube or other body may provide a fluid coupling to establish a fluid pathway between the syringe and the drug mixing device and the same sequence of familiar movements may be performed.

### Staggered Needles

In the embodiment of the present invention discussed above, vial 110 is attached to inner support 150 via needle 310 of the driving fluid transfer member 300 and via needle 230, which forms one end of transfer member 200. When vial 110 is fully inserted into port 106, needles 310 and 230 extend through the opening 110a of vial 110, having previously pierced septum 114 in penetrate into vial 110.

Each of needles 310 and 230 is generally an elongated straight hollow tube and each includes a piercing tip 312, 232 to aid the penetration of the septum 114, and an aperture 314, 234 positioned in the protruding distal end. Straight needles minimise the local hydraulic resistance of the needle.

In each aperture 234, 314, the vector normal to the plane of the aperture is angled with respect to the elongation of the tube of the needle.

Aperture 314 on needle 310 forms an inlet aperture from which driving fluid leaves the driving fluid transfer member 300 and enters the vial 110. Aperture 234 on needle 230 forms an outlet aperture through which the first component of the drug to be mixed 1000 leaves the vial 110 and enters transfer member 200.

One or more of the needles 314, 234 may be made from a polymer. Polymer needles reliably penetrate the septum 114, ensuring adequate fluid coupling and have the advantage that they may be moulded into the inner support 150, streamlining manufacturing. Alternatively, metallic needles, such as stainless steel needles, may be used. Metallic needles reduce fragmentation and coring of the septum during penetration of the septum, and provide rapid equilibration of the fluid being transferred.

Needle 310 protrudes into vial 110 past septum 114 to a greater extent that needle 230, thereby positioning the inlet aperture 314 for the driving fluid further into the vial than the outlet aperture 234 for the first component of the drug to be mixed 1000. In the specific embodiment, needle 310 extends into the vial 110 past septum 114 by 11mm and needle 230 extends into the vial 110 past septum by 9mm, though either of the extensions may lie in the range 1mm to 30mm provided that needle 310 protrudes into vial 110 to a greater extent than needle 230.

When drug mixing device 100 is stood upright on a surface in the configuration of Figure 8, (such as on the ground or on a workbench), the inlet aperture 314 is positioned above the outlet aperture 234, vis-à-vis the ground (as shown in the specific embodiment, inlet aperture 314 is not required to be directly above outlet aperture 234, though it may be). Initially (i.e. prior to any drug mixing) both inlet aperture 314 and outlet aperture 234 are submerged in the first component 1000.

In the specific embodiment, the driving fluid is air, which is less dense than the first component of the drug to be mixed 1000. When the drug mixing device 100 is stood upright and fluid is driven by the fluid driver 600, the less dense driving fluid enters the vial 110 through aperture 314, and forms a bubble of the less dense driving fluid. The bubble rises due to its buoyancy. As a consequence of the location of inlet aperture 314 above the outlet aperture 234, the bubble of less dense driving fluid will never enter aperture 234, thereby avoiding the risk of the driving fluid entering transfer member 200. The accumulation of the less dense driving fluid at the top of vial 110 causes movement of the first component of the drug to be mixed 1000 into the transfer member 200 via outlet aperture 234. Whilst inlet aperture 314 remains submerged in the first component of the drug to be mixed, all bubbles from the inlet aperture 314 will generally rise upwards.

The movement of the first component of the drug to be mixed 1000 into transfer member 200 continues until outlet aperture 234 is no longer submerged. As described above, needle 230, and more specifically outlet aperture 234 of needle 230, is positioned as low within the vial 110 as possible in order to minimise the residual amount of the first component of the drug to be mixed 1000 that is left in the vial 110 when the mixing process by which the first component of the drug to be mixed 1000 is transferred to vial 108 via transfer member 200. Due to the arrangement of the apertures, inlet aperture 314 will always cease to be submerged in the first component of the drug to be mixed before the outlet aperture 234 ceases to be submerged, provided the drug mixing device 100 is stood upright on a surface.

A similar set of staggered needles exists in relation to vial 108, which is attached to inner support 150 via needle 210 of transfer member 200 and needle 410 of exit transfer member 400. Needle 210 forms the other end of transfer member 200 to needle 230. When vial 108 is fully inserted into port 104, needles 210 and 410 extend through the opening 108a of vial 108, having previously pierced septum 112 in penetrate into vial 108, in the configuration shown in Figure 15.

Each of needles 210 and 410 is also generally an elongated straight hollow tube and each includes a piercing tip 212, 412 to aid the penetration of the septum 112, and an aperture 214, 414. Straight needles 210 and 410 also minimise the local hydraulic resistance because they feature no changes of direction. Tips 212, 412 are

Aperture 414 is positioned in the protruding distal end of needle 410 and the vector normal N3 to the plane of the aperture 414 is angled with respect to the elongation of the tube of the needle. Aperture 214 is positioned in the side of needle 210, the vector normal N4 to the plane of aperture 214 is perpendicular to the elongation of the hollow tube (see Figure 15).

Aperture 214 on needle 310 forms an inlet aperture from which the first component of a drug to be mixed 1000 leaves the transfer member 200 and enters the vial 108. Aperture 414 on needle 410 forms an outlet aperture through which, when the drug mixing device is stood upright on a surface, excess air originally present in vial 108 may leave via the exit transfer member 400.

One or more of the needles 210, 410 may be made from a polymer. Polymer needles reliably penetrate the septum 112, ensuring adequate fluid coupling and have the advantage that they may be moulded into the inner support 150, streamlining manufacturing Alternatively, metallic needles, such as stainless steel needles, may be used. Metallic needles reduce fragmentation and coring of the septum during penetration of the septum, and provide rapid equilibration of the fluid being transferred.

Needle 210 protrudes into vial 108 past septum 112 to a greater extent that needle 410, thereby positioning the inlet aperture 214 for the driving fluid further into the vial than the outlet aperture 414 for the first component of the drug to be mixed 1000. In the specific embodiment, needle 210 extends into the vial 108 past septum 112 by 11mm and needle 410 extends into the vial 108 past septum 112 by 9mm. though either of the extensions may lie in the range 1mm to 30mm provided that needle 210 protrudes into vial 110 to a greater extent than needle 410.

The is no specific relationship between the extent to which needles 310 and 230 protrude into vial 110 and the extent to which needles 410 and 210 protrude into vial 108, though for ease of manufacturing, it is possible for needles 310 and 210 to extend into their respective vials by the same amount, and for needles 230 and 410 to extend into their respective vials by the same amount.

When drug mixing device 100 is stood upright on a surface, such as a workbench in the configuration of Figure 8, neither needle 210 nor 410 is submerged, and exit transfer member 400 may contain air originally present in vial 108. However, when the drug mixing device 100 is positioned in the inverted configuration (possibly when part of the fluid transfer assembly 1500 as shown in Figure 13E) after the first and second components of the drug to be mixed 1000, 1010 have mixed to form mixed drug 1020 as previously described, several effects occur.

Inversion of the drug mixing device 100 means that both needles 210 and 410 become submerged in the mixed drug 1020. Inversion also causes the mixed drug 1020 to flow into exit transfer member 400 via outlet aperture 414 in order to prime exit transfer member 400 with mixed drug 1020. Air previously present in the exit transfer member 400 rises to the top of the inverted vial 108. Mixed drug 1020 does not pass back through transfer member 200 because transfer member 200 contains a unidirectional valve to prohibit the flow of mixed drug 1020 from vial 108 to vial 110.

Simultaneously, inversion causes the less dense driving fluid previously accumulated in vial 110 to pass through the outlet aperture 234, through transfer member 200 and into vial 108, because the driving fluid is less dense than the mixed drug 1020. When the less dense driving fluid passes through inlet aperture 214 and into vial 108, a bubble is formed and the bubble rises due to its buoyancy.

As a consequence of the location of inlet aperture 214 above the outlet aperture 414 in the inverted configuration, the bubble of less dense driving fluid will never enter aperture 414, thereby avoiding the risk of the driving fluid (air) entering exit transfer member 400 when the mixed drug 1020 is to be withdrawn from the drug mixing device 100. Instead, there is an accumulation of the less dense driving fluid at the top of vial 108 alongside any air originally present in vial 108, or in exit transfer member 400. Whilst inlet aperture 214 remains submerged in the mixed drug 1020, all bubbles from the inlet aperture 214 will generally rise upwards when the drug mixing device 100 is in the inverted configuration.

With mixed drug 1020 submerging the outlet aperture 414 and entering exit transfer member 400, the mixed drug may be withdrawn from the drug mixing device, for example by a drug administration device such as a syringe 1200. Withdrawal of mixed drug 1020 is possible for as long as the outlet aperture 414 remains submerged.

In similar fashion to needle 230 described above, needle 410, and more specifically outlet aperture 414 of needle 410, is positioned as low within the vial 108 as possible in order to minimise the residual amount of the first component of the drug to be mixed 1000 that is left in the vial 108 when the mixed drug 1020 is withdrawn from the drug mixing device when it is in the inverted configuration. Due to the arrangement of the apertures, inlet aperture 214 will always cease to be submerged in the mixed drug to be mixed before the outlet aperture 414 ceases to be submerged, provided the drug mixing device 100 is in the inverted configuration.

Whilst the preceding describes the specific embodiment of the invention shown in Figures 1 to 20, alternative embodiments of the staggered needles in the drug mixing device 100 exist without departing from the scope of the present invention.

In alternative embodiments, one or more the needles may be metallic, which can provide faster equilibration of the fluid than polymers. The needles need not be elongated straight hollow tubes. Furthermore, the normal vectors N3 to the planes of the apertures may be varied with respect to the elongation of the hollow tube.

In further embodiments, one or more of the needles may initially be protected by a protecting member that covers the apertures prior to use. The protective member advantageously keeps the needle sterile, and prevents a user from needle sticks. The protective member for the one or more needles may be the same protective member that encourages or forces the correct insertion of the vials into the ports of the drug mixing device. Removal of the protective member for the ports thereby simultaneously exposes the needles, speeding up preparation of the drug mixing device.

In alternative embodiments, the inlet aperture and/or the outlet aperture may be positioned on the side of the needles, provided that the relative above/below location of the apertures when the driving fluid enters through the inlet aperture is maintained. Alternative driving fluid, such a nitrogen, might be used, provided they are less dense than the first component of the drug to be mixed.

Whilst in the specific embodiment, the drug mixing device is positioned in an inverted configuration for apertures 214 and 414, a fully inverted configuration is not essential. Partial inversions or other specified orientations are possible in order to avoid the bubbles entering the outlet aperture 414 provided that in such orientations, aperture 214 is above aperture 414 with respect to the ground.

In alternative embodiments, return flow of the mixed drug 1020 to vial 110 may be avoided by means other than a valve, such as by a non-porous membrane.

### Spraying Needle

In the embodiment of the present invention described above, transfer member 200 is fluidly coupled to both vials 108 and 110 via needles 210 and 230 respectively. Aperture 234 formed in needle 230 forms the outlet from vial 110 for the first component of the drug to be mixed 1000 to move from the vial 110 into the transfer member 200. Aperture 214 in needle 210 forms the inlet for vial 108 by which the first component of the drug to be mixed 1000, which flows through the transfer member 200, is dispensed from the transfer member 200 into the vial 108 (as shown in Figure 15). As described above, the dispensing occurs when the drug mixing device 100 is stood upright on its flanged base 103 on a surface, such as a table or a workbench.

Transfer member 200 is a substantially straight tube comprised of needles 210 and 230. Since the transfer member 200 is straight, there are no corners by which cavitation or slack flow may develop as the fluid passes through the transfer member 200 between apertures 234 and 214.

As also described above and shown in Figures 15, 18 and 19A, aperture 214 is disposed in the side of needle 210, adjacent to the distal end of the needle 210. The distal end of the needle 210 is closed. The aperture 214 has a vector normal to the plane of the aperture N4 that is perpendicular, or at least substantially perpendicular to the direction of elongation of the hollow tube of needle 210. Orienting the aperture in this way redirects the first component of the drug to be mixed 1000 that is dispensed from the aperture 214. Prior to dispensing, the first component of the drug to be mixed 1000 has a velocity oriented substantially parallel to the direction of elongation of needle 210. When the drug mixing device 100 is stood upright, this velocity is substantially vertical. As the first component of the drug to be mixed 1000 encounters the aperture 214, the fluid velocity is reoriented to be in the direction of the normal N4 to the aperture 214. In the specific embodiment, the normal to the aperture 214 is horizontal when the drug mixing device 100 is stood upright on its flanged base 103. As such the first component 1000 is fluidly dispensed from the aperture 214 with substantially no vertical component of velocity, and after dispensing through aperture 214, obtains its vertical component velocity due to gravity alone.

Needle 210 extends into vial 108. Vial 108 includes a base 108e and a vial side wall 108f in the main body 108d. Vial side wall 108f forms an inner surface of the vial 108, and base 108e and vial side wall 108f are substantially perpendicular to each other.

Prior to the dispensing of the first component of the drug to be mixed 1000 from aperture 214, the vial side wall 108f has a substantially vertical orientation and the vial base 108e a substantially horizontal orientation, parallel to the flanged base 103 of drug mixing device 100 9 as shown in Figure 15, the vial being placed according to Figures 9 and 10A/B. The second component of the drug to be mixed 1010 hence rests on the base 108e of vial 108 because of gravity (though the second component may also touch the vial side wall 108f at this time).

During dispensing of the fluid (the first component of the drug to be mixed 1000) from the aperture 214, substantially all the fluid leaves the aperture 214 with a velocity that is parallel to the flanged base 103 in the direction N4 as shown in Figure 15. Thereafter, substantially all the fluid dispensed from aperture 214 first encounters the surface of the vial side wall 108f, prior to encountering any other surface of the vial 108. The first encounter with vial side wall 108f is at an oblique angle θ as shown in the inset to Figure 15 (rather than at an angle that is normal to the side wall 108f or to the base 108e). Encountering the surface of the side wall 108f at an oblique angle θ reduces the magnitude of the change in momentum of the particles in the fluid. Reducing the change in momentum of the particles reduces the likelihood of foaming upon encountering the surface of the vial side wall 108f, thereby restricting the agitation of the first component of the drug to be mixed 1000 during the mixing process. The agitation experienced by the fluid particles upon encountering the surface of vial side wall 108f is less than would be experienced if the fluid were dispensed such that it did not encounter the surface at an oblique angle θ (for example, if the fluid were dispensed directly downwards towards the base 108e of vial 108).

Subsequent to the initial encounter with the side wall 108f, the fluid may run down side wall 108f under the action of gravity. The passage of fluid down the side wall 108f further reduces the agitation of the fluid and restricts the formation of foam.

By the above described process, aperture 214 and the surface of vial side wall 108e cooperate to minimise the agitation of the first component of the drug to be mixed 1000 as it is dispensed into the second vial 108. Minimising the agitation of fluid reduces the likelihood of molecules of the first component of the drug to be mixed 1000 being compromised before they have the opportunity to mix with the molecules of the second component of the drug to be mixed 1010.

Whilst the preceding describes the specific embodiment of the invention shown in Figures 1 to 20, alternative embodiments of the spraying needles in the drug mixing device 100 exist without departing from the scope of the present invention.

In the specific embodiment above, the aperture 214 and vial side wall 108f are arranged to reduce agitation and therefore foaming of the first component of the drug to be mixed 1000. However, it is only the relative orientation of the aperture 214 (set by vector N4) and the vial side wall 108e that matters for reducing the momentum change in the initial encounter of the fluid with the side wall 108f. For example, in alternative embodiments, the aperture may be pointed straight downwards, but still encounter the side wall 108f of the vial 108 at an oblique angle θ by orienting the vial 108 (and port 104) to be located at an oblique angle θ to the flanged base 103.

Alternatively or additionally, geometric changes in the transfer member (e.g. funnel shaped tube, a taper, a non-constant diameter etc.) may be used to influence the magnitude of velocity of the first component of the drug to be mixed as it passes through the transfer member 200, and thereby manipulate the magnitude of the velocity be which the first component 1000 is dispensed into vial 108.

In other embodiments, the redirection of the fluid first component of the drug to be mixed 1000 by transfer member 200 may also occur due to a sloped or curved inner wall positioned close to the aperture 214, defined to provide a less abrupt change in velocity of the fluid prior to its dispensing from the aperture 214.

Further reductions in the hydraulic resistance of the transfer member 200 may be made by changing the profile of the aperture 214 in the side of needle 210. For example, the aperture may be bevelled or tapered.

Additional reductions in foaming may be achieved by coating an antifoam agent to at least part of the one or more constituent features of the drug mixing device 100 that encounter the first component of the drug to be mixed 1000. For example, an antifoam agent may be applied to the surface of the vial side wall 108f, or to the transfer member 200, or both. The antifoam agent may be unreactive with the first component of the drug to be mixed 1000, the second component of the drug to be mixed 1010, or with the mixed drug 1020, or a combination thereof.

An antifoam agent may also be coated on at least part of the one or more constituent features of the drug mixing device that encounter the second component of the drug to be mixed 1010, or the mixed drug 1020.

### Transfer Members with Minimised Hydraulic Resistance

The embodiment of the invention described above includes a transfer member 200. As described above, transfer member 200 is fluidly coupled to vial 108 and vial 110 in use and a fluid pathway, through which the first component of the drug to be mixed 1000 may move, exists between vial 110 and vial 108 as a consequence of the fluid coupling provided by transfer member 200. This arrangement is shown in Figure 8.

Transfer member 200 includes two needles 210 and 230 each of which comprises a hollow tube and each of which is oriented in an opposing configuration. The transfer member 200 further includes a hollow tube 220, intermediate the two needles 210 and 230 and fluidly coupled to both needles to form part of the fluid pathway between vials 110 and 108. The overall fluid pathway through transfer member 200 taken by the first component of the drug to be mixed is initially via needle 230, then through tube 230 and finally through needle 210. In alternative configurations, the hollow tube 202 may be omitted and the needles 210 and 230 may be fluidly coupled directly to each other.

In the specific embodiment, transfer member 200 is configured to minimise the hydraulic resistance when the first component of the drug to be mixed 1000 is transferred from vial 110 to vial 108. Transfer member 200, including needles 210, 230 and tube 220 provides a fluid pathway with an overall length of 30mm. However, the fluid pathway may generally fall in the range of 5mm to 100mm and more preferably in the range 5mm to 50mm. Minimising the overall length of the fluid pathway provided by the transfer member 200 minimises the frictional hydraulic resistance experienced by the first component of the drug to mixed 1000 during its passage along the fluid pathway. The length of the fluid pathway may be minimised in part, because of the opposing relationship between vials 110 and 108. As a consequence of the length, less work (provided by the driving fluid) is lost to friction and the mixing process is thereby more efficient.

In addition to the above, transfer member 200 is metallic, specifically stainless steel, in order to reduce the frictional hydraulic resistance because the equilibration of the first component of the drug to be mixed 1000 that is flowing through the transfer member 200 is faster.

The hydraulic resistance provided by transfer member 200 is further minimised by minimising the local hydraulic resistance. In this respect, the transfer member is straight. The straight geometry of the member avoids corners in the fluid pathway that could give rise to regions of cavitation or slack flow.

As described above, vials 108 and 110 are positioned in an opposing relationship (see Figure 8) about the inner support 150, attached via needles 210, 230, 310 and 410. When drug mixing device 100 is stood upright on the flanged base 103, in addition to having movement of the first component of the drug to be mixed 1000 from the vial 110 to the vial 108 as a consequence of a pressure gradient, the movement of the first component 1000 is also is gravitationally assisted. Gravitational assistance reduces the work required in order to cause movement of the first component 1000 into the vial 108 during the mixing process.

As described above, transfer member 200 may contain valve, such as a one-way valve, to restrict the direction of flow and prevent return flow from vial 108 to vial 110 when the device is reoriented or when the pressure gradient would favour such return flow.

By the above features of the transfer member 200, the hydraulic resistance of the transfer member is reduced, resulting in less work being required in order to move the first component of the drug to be mixed 1000 from vial 110 to the vial 108. Furthermore, the work required is further reduced by the opposing relationship between the vials, which enables gravity to assist in the movement of the first component 1000.

The above features (and the alternatives described below), whilst described in conjunction with transfer member 200, may nevertheless be provided in the driving fluid transfer member 300 for minimising the hydraulic resistance to the movement of the driving fluid, and/or the exit transfer member 400 to minimise the hydraulic resistance to the movement of the mixed drug 1020, as appropriate.

Whilst the preceding describes the specific embodiment of the invention shown in Figures 1 to 20, alternative embodiments of the transfer member of the drug mixing device 100 exist without departing from the scope of the present invention.

In alternative embodiments, the hydraulic resistance is also to be minimized for movement of the second component of the drug to be mixed 1010.

In further alternative embodiments, the transfer member is made of a different metal other than stainless steel. The transfer member may also be made of a polymer. Polymer needles are particularly reliable for use in transfer members, and are not easily damaged.

In further alternative embodiments, the transfer member may also incorporate a friction reducing coating, such as polytetrafluoroethene, a silicone coating or a siliconized coating. The friction reducing coating further reduces the frictional component of the hydraulic resistance. In some alternative embodiments, the friction reducing component is unreactive with one or more of the first component 1000, second component 1010 and the mixed drug 1020.

Additionally or alternatively, further adaptations of the transfer member 200 that enable a reduction in hydraulic resistance may be included in the embodiments. For example, either of the inlet aperture 234 or the outlet aperture 24 may include a geometry to minimise the hydraulic resistance. For example, one or other of the apertures may be bevelled to reduce the local hydraulic resistance of the aperture because no sharp edges are present. As an alternative example, one or other of the apertures may be tapering contrary to the direction of the movement of the first component 1000 in order to increase the diameter of the aperture and reduce the frictional hydraulic resistance. Both apertures may include one, or both of the above adaptations. These examples are shown in Figures 19B and 19D, alongside a straight-edged example in Figure 19C.

### Gravitational Locking Mechanism

As described in the push and forget section above, the embodiment of the invention shown in Figures 1 to 20 features a locking mechanism 520 as part of actuator 500. The locking mechanism transitions from a locked state to and unlocked state upon removal of the pin 534 as is described above. In an alternative embodiment, actuator 500 is replaced by actuator 500'. Similarly to actuator 500, actuator 500' is configured to respond to trigger 550 and, provided the actuator 500' is not in a locked state, actuator 500' interfaces with fluid driver 600 to cause mixing of the drug. Actuator 500' hence couples the trigger 550 to the fluid driver 600.

Actuator 500' is substantially similar to actuator 500. Actuator 500' includes gravitational locking mechanism 820, as shown in figures 21 and 22, which can be incorporated into actuator 500' independently of, or in combination with, locking mechanism 520. Actuation of actuator 500' is prevented when gravitational locking mechanism 820 is in the locked state, as shown in figures 21A and 22A, and permitted when gravitational locking mechanism 820 is in the unlocked state, as shown in figures 21B and 22B. Gravitational locking mechanism 820 is configured to adopt the unlocked state only when drug mixing device 100 is oriented in a specific orientation which, in the exemplary embodiments discussed herein, corresponds to the drug mixing device being stood upright on flanged base 103. Gravitational locking mechanism 820 is configured such that transition of the gravitational locking mechanism between the locked and unlocked states occurs by virtue of the influence of gravity. Preventing mixing of the drug when drug mixing device 100 is not oriented in the specific orientation provides various benefits, such as improving stability of the device while mixing occurs, and the opportunity for mixing to be assisted by gravity, as discussed in the housing and structure, pressure driven mixing, and transfer members with minimised hydraulic resistance sections above.

In a specific embodiment, similar to that discussed in the push and forget section above and shown in Figure 16, gravitational locking mechanism 820 includes a substantially circular ring 822 disposed in slot around a portion of actuator 500'. Ring 822 includes protrusions 824, 826, 828 (which are similar to protrusions 524, 526 and 528) and 830, each of which extend radially outwards from diametrically opposed locations on the ring in a 'cross' configuration. These protrusions are initially in a first position where actuation of the actuator 500' is prohibited, as discussed in the push and forget section above. Protrusion 830 may be substantially similar to arm 530 with hole 532 as described in the push and forget section above, and shown in Figure 8.

The underside of button 552 includes four camming surfaces 554, 556, 558 and 560. Each camming surface is configured to interface with one of protrusions 824, 826, 828 or 830. Each of the camming surfaces is configured to turn translational depressive movement of button 552 into a rotational movement of circular ring 822. Gravitational locking mechanism 820 in the locked state prevents rotational movement of circular ring 822, thereby preventing movement of the protrusions from a first position where actuation of actuator 500' is prohibited, to a second position where actuation of actuator 500' is permitted.

Gravitational locking mechanism 820 comprises a first part 840 and a second part 850, which cooperate with each other to place the gravitational locking mechanism in the locked and unlocked states. In an exemplary embodiment, the first part is a ball and the second part is a socket.

In one exemplary embodiment of gravitational locking mechanism 820, illustrated by figures 21A and 21B, circular ring 822 is formed with a socket 850 in its underside which is sized to receive between half and three quarters of the ball 840. A recess 842 is formed in a rotationally fixed part of drug mixing device 100, such as piston 604, which is located directly beneath socket 850 (when the drug mixing device is assembled) and oriented in the specific orientation. The recess 842 is sized to receive the entirety of ball 840. Recess 842 may be of any suitable shape, provided that if the orientation of drug mixing device is altered from the specific orientation to an alternate orientation, ball 840 rolls or slides out of recess 842 and reengages with socket 850.

When ball 840 resides at least partly within socket 850, due to drug mixing device 100 not being oriented in the specific orientation, the first and second parts are coupled, and gravitational locking mechanism 820 is in the locked state, as shown in figure 21A. As the recess 842 is formed in a rotationally fixed component of the drug mixing device, when the ball 840 and socket 850 are coupled, circular ring 822 cannot rotate to move the protrusions from a first position where actuation of actuator 500' is prohibited, to a second position where actuation of actuator 500' is permitted.

When drug mixing device 100 is oriented in the specific orientation, gravitational locking mechanism 820 adopts the unlocked state, as shown in figure 21B. In this adoption of the unlocked state, ball 840 translates such that it is received entirely by recess 842 and decoupled from socket 850, allowing circular ring 822 to rotate, provided that any other locking mechanisms are also in their unlocked states, when camming of the protrusions 824, 826, 828 and 830 is initiated by translational depressive movement of button 552. This rotary movement moves the protrusions from a first position where actuation of actuator 500' is prohibited, to a second position where actuation of actuator 500' is permitted

The socket 850 being sized to receive at least half of ball 840 acts to prevent the ball from translating into recess 842, and therefore causing gravitational locking mechanism 822 to adopt the unlocked state, if rotational force is applied to the protrusions 824, 826, 828 and 830 of circular ring 822 when drug mixing device 100 is not oriented in the specific orientation.

In an alternative embodiment of the gravitational locking mechanism 822, the recess 842, sized to receive the ball 840 entirely, may be located in the circular ring 822, and the socket 850, sized to receive at least half of the ball 840, may be located in a rotationally fixed component of the drug mixing device, such as the piston 604, above the circular ring 822 with respect to the flanged base 103.

In an alternative embodiment of the gravitational locking mechanism 822, the first part 840 and the second part are coupled when the gravitational locking mechanism is in the unlocked state, as shown in figure 22B.

In an exemplary embodiment of this configuration, illustrated by figures 22A and 22B, circular ring 822 is comprised of upper and lower circular rings 822a and 822b. Upper circular ring 822a is disposed above lower circular ring 822b when drug mixing device 100 is oriented with flanged base 103 at the bottom. Upper circular ring 822a includes protrusions 824a, 826a, 828a and 830a, and lower circular ring 822b includes protrusions 824b, 826b, 828b and 830b. Corresponding a and b protrusions align and, in combination, form similar protrusions to protrusions 524, 526, 528 and 530, and 824, 826, 828 and 830 which are discussed above.

Upper circular ring 822a is formed with a recess 842 in its underside which is sized to receive the entirety of ball 840. A socket 850 is formed in the upper side of 822b, and is sized to receive between half and three quarters of ball 840. Camming surfaces 554, 556, 558 and 560 of button 552 are configured to interface with protrusions 824a, 826a, 828a and 830a, and are configured to turn translational depressive movement of button 552 into a rotational movement of upper circular ring 822a.

When drug mixing device 100 is oriented in the specific orientation, ball 840 is located partially in socket 850. Hence, upper circular ring 822a and lower circular ring 822b are coupled and gravitational locking mechanism 822 is in the unlocked state, as shown in figure 22B. In this unlocked state, rotation of upper circular ring 822a causes corresponding rotation of lower circular ring 822b. This coupled rotation of the upper and lower circular rings moves all of the protrusions (824a, 824b, 826a, 826b, 828a, 828b, 830a and 830) from a first position where actuation of actuator 500' is prohibited, to a second position where actuation of actuator 500' is permitted.

When drug mixing device 100 is not oriented in the specific orientation, ball 840 is located entirely in recess 842 and uncoupled from socket 850, therefore upper circular ring 822a and lower circular ring 822b are uncoupled and gravitational locking mechanism 822 is in the locked state, as shown in figure 22A. In this locked state, translational depressive movement of the button 552 and subsequent rotation of upper circular ring 822a does not cause rotation of lower circular ring 822b, and therefore does not cause movement of protrusions 824b, 826b, 828b and 830b from a first position where actuation of actuator 500' is prohibited, to a second position where actuation of actuator 500' is permitted.

Actuator 500' of this embodiment may further include a mechanism, such as an elastic member, to facilitate or cause the return of upper circular ring 822a into alignment with lower circular ring 822b, should rotation of upper circular ring 822 occur while rings are not coupled.

In an alternative embodiment, circular ring 822b may be omitted, and piston 604 may formed with protrusions 824b, 826b, 828b and 830b and socket 850, and be rotatable about the same axis as upper circular ring 822a

Whilst the preceding describes the specific embodiment of the invention shown in Figures 21A to 22B, alternative embodiments of the actuator of the drug mixing device 100 exist without departing from the scope of the present invention.

In alternative embodiments, the first and second parts may not be a ball and socket, for example the first part could be an elongated rod.

In alternative embodiments, the first and second parts of the gravitational locking mechanism may be formed or located in one or more of the protrusions of the circular ring and/or in corresponding fixed parts of the housing, or otherwise, of the drug mixing device.

## Claims

1. A drug mixing device comprising:
a movable actuator (500), wherein movement of the actuator causes mixing of a drug within the drug mixing device; and
an elastic member (710) coupled to the actuator, the elastic member configured upon release to transition from an extended state to a non-extended state, the elastic member storing energy in the extended state,
wherein the movement of the actuator is driven by the release of the stored energy as the elastic member transitions from the extended state towards the non-extended state.

2. The mixing device of claim 1, wherein the movement of the actuator causes movement of a first component (1000) of the drug to be mixed from a first portion of the drug mixing device to a second portion of the drug mixing device where it is mixed with a second component (1010) to form the mixed drug (1020),
optionally wherein the drug mixing device is for reconstitution of the drug, further optionally wherein the drug is Remicade (RTM).

3. The mixing device of claim 1 or claim 2 wherein the drug mixing device further comprises a fluid driver (600).

4. The mixing device of claim 3, wherein the fluid driver comprises a movable piston (604) and a driving fluid container (602)

5. The mixing device of claim 4, wherein the container comprises a container of driving fluid, wherein movement of the piston causes at least part of the driving fluid to be expelled from the driving fluid container,
optionally wherein the volume of driving fluid expelled from the driving fluid container lies between 1ml and 20ml.

6. The mixing device of any one of the preceding claims, wherein the elastic member comprises a spring.

7. The mixing device of claim 6, wherein the spring comprises a flat spring.

8. The mixing device of claim 6, wherein the spring comprises a coil spring.

9. The mixing device of any one of claims 6 to 8, wherein the spring comprises an extended portion, the extended portion (710a) arranged to substantially conform to an external contour of at least a portion of the actuator.

10. The mixing device of claim 8 wherein the coil spring is in part or in its entirety wrapped around at least a portion of the actuator.

11. The mixing device of claim 8 when dependent on claim 4, wherein the coil spring is in part or its entirety wrapped around the driving fluid container.

12. The mixing device of any one of the preceding claims, wherein the movement of the actuator is directed substantially towards a point to which the elastic member is secured.

13. The mixing device of any one of the preceding claims, further comprising a locking mechanism (520) configured in a locked state to prevent the release of energy from the elastic member, and in an unlocked state to allow the release of energy from the elastic member.

14. The mixing device of any one of the preceding claims, further comprising a housing (101), wherein the housing is configured to detachably receive within a first portion of the housing a first container (108) for holding a first component of the drug to be mixed, and detachably receive within a second portion of the housing a second container (110) for holding a second component of the drug to be mixed.

15. The mixing device of claim 14, wherein the volume of at least one of the first container and the second container is in the range 1ml to 1000ml.

## Patentansprüche

1. Vorrichtung zum Mischen von Arzneimitteln, umfassend:
ein bewegliches Stellglied (500), wobei die Bewegung des Stellglieds das Mischen eines Arzneimittels im Innern der Vorrichtung zum Mischen von Arzneimitteln veranlasst; und
ein elastisches Element (710), das mit dem Stellglied gekoppelt ist, wobei das elastische Element dazu ausgelegt ist, bei Freisetzung aus einem ausgefahrenen Zustand in einen nicht ausgefahrenen Zustand überzugehen, wobei das elastische Element in dem ausgefahrenen Zustand Energie speichert,
wobei die Bewegung des Stellglieds durch die Freisetzung der gespeicherten Energie angetrieben wird, wenn das elastische Element aus dem ausgefahrenen Zustand in den nicht ausgefahrenen Zustand übergeht.

2. Vorrichtung zum Mischen nach Anspruch 1, wobei die Bewegung des Stellglieds die Bewegung einer ersten Komponente (1000) des zu mischenden Arzneimittels aus einem ersten Abschnitt der Vorrichtung zum Mischen von Arzneimitteln in einen zweiten Abschnitt der Vorrichtung zum Mischen von Arzneimitteln veranlasst, wo sie mit einer zweiten Komponente (1010) gemischt wird, um das gemischte Arzneimittel (1020) zu bilden, wobei optional die Vorrichtung zum Mischen von Arzneimitteln der Rekonstitution des Arzneimittels dient, wobei ferner optional das Arzneimittel Remicade (RTM) ist.

3. Vorrichtung zum Mischen nach Anspruch 1 oder Anspruch 2, wobei die Vorrichtung zum Mischen von Arzneimitteln einen Fluidtreiber (600) umfasst.

4. Vorrichtung zum Mischen nach Anspruch 3, wobei der Fluidtreiber einen beweglichen Kolben (604) und einen Antriebsfluidbehälter (602) umfasst.

5. Vorrichtung zum Mischen nach Anspruch 4, wobei der Behälter einen Behälter mit Antriebsfluid umfasst, wobei die Bewegung des Kolbens veranlasst, dass zumindest ein Teil des Antriebsfluids aus dem Antriebsfluidbehälter ausgestoßen wird,
wobei optional das Volumen des Antriebsfluids, das aus dem Antriebsfluidbehälter ausgestoßen wird, zwischen 1 ml und 20 ml beträgt.

6. Vorrichtung zum Mischen nach einem der vorhergehenden Ansprüche, wobei das elastische Element eine Feder umfasst.

7. Vorrichtung zum Mischen nach Anspruch 6, wobei die Feder eine flache Feder umfasst.

8. Vorrichtung zum Mischen nach Anspruch 6, wobei die Feder eine Spiralfeder umfasst.

9. Vorrichtung zum Mischen nach einem der Ansprüche 6 bis 8, wobei die Feder einen verlängerten Abschnitt umfasst, wobei der verlängerte Abschnitt (710a) angeordnet ist, um im Wesentlichen einer Außenkontur von mindestens einem Abschnitt des Stellglieds zu entsprechen.

10. Vorrichtung zum Mischen nach Anspruch 8, wobei die Spiralfeder ganz oder teilweise um mindestens einen Abschnitt des Stellglieds gewickelt ist.

11. Vorrichtung zum Mischen nach Anspruch 8 in Abhängigkeit von Anspruch 4, wobei die Spiralfeder ganz oder teilweise um den Antriebsfluidbehälter gewickelt ist.

12. Vorrichtung zum Mischen nach einem der vorhergehenden Ansprüche, wobei die Bewegung des Stellglieds im Wesentlichen auf einen Punkt gerichtet ist, an dem das elastische Element befestigt ist.

13. Vorrichtung zum Mischen nach einem der vorhergehenden Ansprüche, ferner umfassend einen Verriegelungsmechanismus (520), der dazu ausgelegt ist, in einem verriegelten Zustand die Freisetzung von Energie aus dem elastischen Element zu verhindern und in einem nicht verriegelten Zustand die Freisetzung von Energie aus dem elastischen Element zu erlauben.

14. Vorrichtung zum Mischen nach einem der vorhergehenden Ansprüche, ferner umfassend ein Gehäuse (101), wobei das Gehäuse dazu ausgelegt ist, in einem ersten Abschnitt des Gehäuses einen ersten Behälter (108) zur Aufnahme einer ersten Komponente des zu mischenden Arzneimittels lösbar aufzunehmen und in einem zweiten Abschnitt des Gehäuses einen zweiten Behälter (110) zur Aufnahme einer zweiten Komponente des zu mischenden Arzneimittels lösbar aufzunehmen.

15. Vorrichtung zum Mischen nach Anspruch 14, wobei das Volumen von mindestens einem des ersten Behälters und des zweiten Behälters im Bereich von 1 ml bis 1000 ml liegt.

## Revendications

1. Dispositif de mélange de médicament, comprenant :
un actionneur déplaçable (500), le déplacement de l'actionneur provoquant le mélange d'un médicament à l'intérieur du dispositif de mélange de médicament ; et
un organe élastique (710) accouplé à l'actionneur, l'organe élastique étant configuré, lors de sa libération, pour passer d'un état étiré à un état non étiré, l'organe élastique stockant de l'énergie dans l'état étiré,
le déplacement de l'actionneur étant entraîné par la libération de l'énergie stockée lorsque l'organe élastique passe de l'état étiré à l'état non étiré.

2. Dispositif de mélange selon la revendication 1, dans lequel le déplacement de l'actionneur provoque le déplacement d'un premier composant (1000) du médicament devant être mélangé d'une première portion du dispositif de mélange de médicament à une deuxième portion du dispositif de mélange de médicament dans laquelle il est mélangé avec un deuxième composant (1010) pour former le médicament mélangé (1020), facultativement, dans lequel le dispositif de mélange de médicament est prévu pour reconstituer le médicament, et facultativement en outre dans lequel le médicament est le Remicade (RTM).

3. Dispositif de mélange selon la revendication 1 ou la revendication 2, le dispositif de mélange de médicament comprenant en outre un dispositif d'entraînement de fluide (600).

4. Dispositif de mélange selon la revendication 3, dans lequel le dispositif d'entraînement de fluide comprend un piston déplaçable (604) et un récipient de fluide d'entraînement (602).

5. Dispositif de mélange selon la revendication 4, dans lequel le récipient comprend un récipient de fluide d'entraînement, le déplacement du piston provoquant l'expulsion d'au moins une partie du fluide d'entraînement hors du récipient de fluide d'entraînement,
facultativement, dans lequel le volume de fluide d'entraînement expulsé du récipient de fluide d'entraînement est compris entre 1 ml et 20 ml.

6. Dispositif de mélange selon l'une quelconque des revendications précédentes, dans lequel l'organe élastique comprend un ressort.

7. Dispositif de mélange selon la revendication 6, dans lequel le ressort comprend un ressort plat.

8. Dispositif de mélange selon la revendication 6, dans lequel le ressort comprend un ressort enroulé.

9. Dispositif de mélange selon l'une quelconque des revendications 6 à 8, dans lequel le ressort comprend une portion étirée, la portion étirée (710a) étant prévue pour épouser sensiblement la forme d'un contour extérieur d'au moins une portion de l'actionneur.

10. Dispositif de mélange selon la revendication 8, dans lequel le ressort enroulé est enveloppé en partie ou en totalité autour d'au moins une portion de l'actionneur.

11. Dispositif de mélange selon la revendication 8, lorsqu'elle dépend de la revendication 4, dans lequel le ressort enroulé est enveloppé en partie ou en totalité autour du récipient de fluide d'entraînement.

12. Dispositif de mélange selon l'une quelconque des revendications précédentes, dans lequel le déplacement de l'actionneur est orienté sensiblement vers un point auquel est fixé l'organe élastique.

13. Dispositif de mélange selon l'une quelconque des revendications précédentes, comprenant en outre un mécanisme de verrouillage (520) configuré, dans un état verrouillé, pour empêcher la libération d'énergie de l'organe élastique, et, dans un état déverrouillé, pour permettre la libération d'énergie de l'organe élastique.

14. Dispositif de mélange selon l'une quelconque des revendications précédentes, comprenant en outre un boîtier (101), le boîtier étant configuré pour recevoir de manière détachable à l'intérieur d'une première portion du boîtier un premier récipient (108) pour contenir un premier composant du médicament devant être mélangé, et pour recevoir de manière détachable à l'intérieur d'une deuxième portion du boîtier un deuxième récipient (110) pour retenir un deuxième composant du médicament devant être mélangé.

15. Dispositif de mélange selon la revendication 14, dans lequel le volume d'au moins l'un parmi le premier récipient et le deuxième récipient est dans une plage de 1 ml à 1000 ml.
